(19) Europäisches Patentamt / European Patent Office / Office européen des brevets

(11) **EP 3 806 028 B1**

(12) **EUROPEAN PATENT SPECIFICATION**

(45) Date of publication and mention
of the grant of the patent:
**20.12.2023 Bulletin 2023/51**

(21) Application number: **19808389.1**

(22) Date of filing: **11.04.2019**

(51) International Patent Classification (IPC):
**G06T 5/00** *(2006.01)*

(52) Cooperative Patent Classification (CPC):
**G01N 29/0645; A61B 8/483; G01N 29/0609;**
**G01S 15/8977; G01S 15/8993; G06T 15/00;**
A61B 8/466; G01N 2291/02475; G01S 15/8915

(86) International application number:
**PCT/CN2019/082216**

(87) International publication number:
**WO 2019/223442 (28.11.2019 Gazette 2019/48)**

(54) **ULTRASOUND IMAGE DISPLAY METHOD AND APPARATUS, AND STORAGE MEDIUM AND ELECTRONIC APPARATUS**

VERFAHREN UND VORRICHTUNG ZUR ANZEIGE VON ULTRASCHALLBILDERN SOWIE SPEICHERMEDIUM UND ELEKTRONISCHE VORRICHTUNG

PROCÉDÉ ET APPAREIL D'AFFICHAGE D'IMAGE ULTRASONORE, SUPPORT D'INFORMATIONS ET APPAREIL ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2018 CN 201810508663**

(43) Date of publication of application:
**14.04.2021 Bulletin 2021/15**

(73) Proprietor: **Tencent Technology (Shenzhen) Company Limited**
**Shenzhen, Guangdong, 518057 (CN)**

(72) Inventor: **WANG, Liang**
**Shenzhen, Guangdong 518057 (CN)**

(74) Representative: **Eisenführ Speiser**
**Patentanwälte Rechtsanwälte PartGmbB**
**Postfach 31 02 60**
**80102 München (DE)**

(56) References cited:
**CN-A- 101 061 961      CN-A- 101 061 961**

**CN-A- 103 512 960      CN-A- 103 512 960**
**JP-A- 2005 087 237      US-A1- 2016 238 568**

• **WANG LIANG ET AL: "3-D biquaternionic analytic signal and application to envelope detection in 3-D ultrasound imaging", 2012 INTERNATIONAL CONFERENCE ON 3D IMAGING (IC3D), IEEE, 3 December 2012 (2012-12-03), pages 1-8, XP032491676, DOI: 10.1109/IC3D.2012.6615144 [retrieved on 2013-09-27]**
• **KAJETANA M SNOPEK: "The n-D analytic signals and Fourier spectra in complex and hypercomplex domains", TELECOMMUNICATIONS AND SIGNAL PROCESSING (TSP), 2011 34TH INTERNATIONAL CONFERENCE ON, IEEE, 18 August 2011 (2011-08-18), pages 423-427, XP031975172, DOI: 10.1109/TSP.2011.6043697 ISBN: 978-1-4577-1410-8**
• **THOMAS BULOW ET AL.: "Hypercomplex Signals-A Novel Extension of the Analytic Signal to the Multidimensional Case", IEEE TRANSACTIONS ON SIGNAL PROCESSING, IEEE SERVICE CENTER, NEW YORK, NY, US, vol. 49, no. 11, 1 November 2001 (2001-11-01), pages 2844-2852, XP011059444, ISSN: 1053-587X**

## Description

This application claims priority to Chinese Patent Application No. 201810508663.2, entitled "ULTRASOUND IMAGE DISPLAY METHOD AND APPARATUS, AND STORAGE MEDIUM AND ELECTRONIC APPARATUS" and filed with the National Intellectual Property Administration, PRC on May 24, 2018.

## TECHNICAL FIELD

This application relates to the field of computers, and in particular to display of an ultrasound image.

## BACKGROUND

Envelope detection is an important step of reconstruction of a brightness-mode ultrasound image (B-mode ultrasound image for short). A basic process of the reconstruction of the B-mode ultrasound image includes: acquiring a high-frequency radio frequency (RF for short) signal from an ultrasound probe, the original RF signal being a one-dimensional signal along a direction of the ultrasound probe; performing the Hilbert transform on the one-dimensional signal to construct a one-dimensional analytic signal; obtaining an amplitude value of the one-dimensional analytic signal as a one-dimensional envelope signal; splicing multiple one-dimensional envelope signals according to locations of the probe, to acquire a two-dimensional envelope image; and acquiring a two-dimensional B-mode ultrasound image by using some post-processing. At present, a three-dimensional B-mode ultrasound image is generally acquired by splicing one-dimensional envelope signals to acquire a three-dimensional envelope image, and performing post-processing on the three-dimensional envelope image.

L. Wang et al., "3-D biquaternionic analytic signal and application to envelope detection in 3-D ultrasound imaging", IEEE International Conference on 3D Imaging, 2012, gives an algebraic framework for 3-D analytic signals and realizes a numerical implementation of Clifford Fourier transformation and Clifford biquaternions. An input signal, which is a three-dimensional radio-frequency signal, is obtained by performing detection on an object to be detected, and a hypercomplex analytic signal is acquired, corresponding to the input signal. The amplitude of said analytic signal is calculated from the partial module of its biquaternionic representation and is used as envelope information to be displayed in the 3-D ultrasound data.

US 2016/238568 A1 discloses an ultrasonic material-evaluation or classification method using spectral and envelope-statistics variables from back-scattered ultrasound echo signals combined with global variables.

CN 101 061 961 A relates to an ultrasonic image acquisition device and an ultrasonic image acquisition method which perform three-dimensional scanning, and particularly relates to a technology for adjusting the brightness of an ultrasonic image.

CN 103 512 960 A relates to an array of ultrasonic nondestructive testing technology, particularly to a method of using an ultrasound imaging array.

K.M. Snopek, "The n-D Analytic Signals and Fourier Spectra in Complex and Hypercomplex Domains", IEEE International Conference on Telecommunications and Signal Processing, 2011, investigates two various representations of a n-dimensional (n-D) real signal, such as used for instance in colour image processing. The first one is the n-D complex analytic signal with a single-orthant spectrum defined as the extension of the 1-D Gabor's analytic signal. The second one is the n-D hypercomplex analytic signal defined in Clifford and Cayley-Dickson algebras.

T. Bulow et al., "Hypercomplex Signals-A Novel Extension of the Analytic Signal to the Multidimensional Case", IEEE Transactions on Signal Processing, 2001, proposes a hypercomplex signal as a novel extension of a complex signal to n-dimensions, with possible applications in image processing.

## SUMMARY

A method and an apparatus for displaying an ultrasound image, a storage medium and an electronic device are provided according to embodiments of the present disclosure, to improve the accuracy of a three-dimensional B-mode ultrasound image.

According to an aspect of the present invention, a method for displaying an ultrasound image is provided, including: acquiring an input signal obtained by performing detection on a to-be-detected object by a detection device, the input signal being a three-dimensional radio-frequency signal; performing a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and displaying the envelope information in the three-dimensional ultrasound image, the envelope information being used for indicating the to-be-detected object, wherein the performing a one-time modulus value calculation on the three-dimensional radio-frequency signal comprises:

acquiring a first hypercomplex signal corresponding to the three-dimensional radio-frequency signal, the first hypercomplex signal being a sum of 8 components, and each of the 8 components being represented by modulus values and angles of a plurality of analytic signals corresponding to the input signal; and

acquiring the envelope information comprising a modulus value of the first hypercomplex signal, the modulus value of the first hypercomplex signal being used for representing the three-dimensional amplitude of the three-dimensional radio-frequency signal,

wherein the modulus value of the first hypercomplex signal is acquired according to following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

$|\psi_{cas}|$ representing the modulus value of the first hypercomplex signal, $\alpha_1$ being a modulus value of a first analytic signal, $\varphi_1$ being an angle of the first analytic signal, the first analytic signal being an analytic signal that is in a first orthant of 8 orthants in a three-dimensional frequency domain and that corresponds to the input signal, $\alpha_3$ being a modulus value of a third analytic signal, $\varphi_3$ being an angle of the third analytic signal, the third analytic signal being an analytic signal that is in a third orthant of the 8 orthants in the three-dimensional frequency domain and that corresponds to the input signal, $\alpha_5$ being a modulus value of a fifth analytic signal, $\varphi_5$ being an angle of the fifth analytic signal, the fifth analytic signal being an analytic signal that is in a fifth orthant of the 8 orthants in the three-dimensional frequency domain and that corresponds to the input signal, $\alpha_7$ being a modulus value of a seventh analytic signal, $\varphi_7$ being an angle of the seventh analytic signal, the seventh analytic signal being an analytic signal that is in a seventh orthant of the 8 orthants in the three-dimensional frequency domain and that corresponds to the input signal, and the plurality of analytic signals comprising the first analytic signal, the third analytic signal, the fifth analytic signal, and the seventh analytic signal.

According to another aspect of the present invention, an apparatus for displaying an ultrasound image is further provided, including: an acquiring unit, configured to acquire an input signal obtained by performing detection on a to-be-detected object by a detection device, the input signal being a three-dimensional radio-frequency signal; a calculating unit, configured to perform a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and a display unit, configured to display the envelope information in the three-dimensional ultrasound image, the envelope information being used for indicating the to-be-detected object, wherein the calculating unit comprises:

a first acquiring module, configured to acquire a first hypercomplex signal corresponding to the three-dimensional radio-frequency signal, the first hypercomplex signal being a sum of 8 components, and each of the 8 components being represented by modulus values and angles of a plurality of analytic signals corresponding to the input signal; and

a second acquiring module, configured to acquire the envelope information comprising a modulus value of the first hypercomplex signal, the modulus value of the first hypercomplex signal being used for representing the three-dimensional amplitude of the three-dimensional radio-frequency signal,

the second acquiring module being configured to acquire the modulus value of the first hypercomplex signal according to following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

$|\psi_{cas}|$ representing the modulus value of the first hypercomplex signal, $\alpha_1$ being a modulus value of a first analytic signal, $\varphi_1$ being an angle of the first analytic signal, the first analytic signal being an analytic signal that is in a first orthant of 8 orthants in a three-dimensional frequency domain and that corresponds to the input signal, $\alpha_3$ being a modulus value of a third analytic signal, $\varphi_3$ being an angle of the third analytic signal, the third analytic signal being an analytic signal that is in a third orthant of the 8 orthants in the three-dimensional frequency domain and that corresponds to the input signal, $\alpha_5$ being a modulus value of a fifth analytic signal, $\varphi_5$ being an angle of the fifth analytic signal, the fifth analytic signal being an analytic signal that is in a fifth orthant of the 8 orthants in the three-dimensional frequency domain and that corresponds to the input signal, $\alpha_7$ being a modulus value of a seventh analytic signal, $\varphi_7$ being an angle of the seventh analytic signal, the seventh analytic signal being an analytic signal that is in a seventh orthant of the 8 orthants in the three-dimensional frequency domain and that corresponds to the input signal, and the plurality of analytic signals comprising the first analytic signal, the third analytic signal, the fifth analytic signal, and the seventh analytic signal.

## BRIEF DESCRIPTION OF THE DRAWINGS

The accompanying drawings described herein are used for providing a further understanding of the present disclosure, and form a part of the present disclosure. Exemplary embodiments of the present disclosure and descriptions thereof are used for explaining the present disclosure, and do not constitute any inappropriate limitation to the present disclosure. In the accompanying drawings:

FIG. 1 is a schematic diagram of a hardware environment of a method for displaying an ultrasound image according to an embodiment of the present disclosure;

FIG. 2 is a flowchart of a method for displaying an ultrasound image according to an optional embodiment of the present disclosure;

FIG. 3 is a schematic diagram of forming a plane by splicing envelopes of one-dimensional signals in the related art;

FIG. 4 is a schematic diagram of 8 orthants in a 3D frequency domain according to an embodiment of the present disclosure;

FIG. 5 is a schematic diagram of a platform where a three-dimensional radio-frequency signal is collected according to an embodiment of the present disclosure;

FIG. 6 is a structural diagram of a three-dimensional radio-frequency signal according to an embodiment of the present disclosure;

FIG. 7 is a comparison diagram of an envelope image in a three-dimensional ultrasound image according to an embodiment of the present disclosure;

FIG. 8 is an enlarged schematic diagram of an envelope image in a three-dimensional ultrasound image according to an embodiment of the present disclosure;

FIG. 9 is an enlarged schematic diagram of a brightness comparison in the vertical direction according to an embodiment of the present disclosure;

FIG. 10 is an enlarged schematic diagram of a brightness comparison in the biopsy needle direction according to an embodiment of the present disclosure;

FIG. 11 is a schematic diagram of a platform where a three-dimensional radio-frequency signal is collected by a linear probe according to an embodiment of the present disclosure;

FIG. 12 is a structural diagram of a three-dimensional radio-frequency signal collected by a linear probe according to an embodiment of the present disclosure;

FIG. 13 is a comparison diagram of an envelope image in a three-dimensional ultrasound image collected by a linear probe according to an embodiment of the present disclosure;

FIG. 14 is a schematic diagram of a brightness comparison of an envelope image in the vertical direction in a three-dimensional ultrasound image collected by a linear probe according to an embodiment of the present disclosure;

FIG. 15 is a schematic diagram of a brightness comparison of an envelope image in the biopsy needle direction in a three-dimensional ultrasound image collected by a linear probe according to an embodiment of the present disclosure;

FIG. 16 is a schematic diagram of an apparatus for displaying an ultrasound image according to an optional embodiment of the present disclosure; and

FIG. 17 is a block diagram showing a structure of an electronic device according to an embodiment of the present disclosure.

## DESCRIPTION OF EMBODIMENTS

To make solutions of the present disclosure more comprehensible for a person skilled in the art, the following clearly and completely describes the technical solutions in the embodiments of the present disclosure with reference to the accompanying drawings in the embodiments of the present disclosure. Apparently, the described embodiments are merely a part rather than all of the embodiments of the present disclosure.

The terms such as "first" and "second" in the specification, claims, and accompanying drawings of the present disclosure are intended to distinguish between similar objects rather than describe a particular sequence or a chronological order. It is to be understood that the data termed in such a way are interchangeable in proper circumstances so that the embodiments of the present disclosure described herein can be implemented in orders except the order illustrated or described herein. In addition, the terms "include", "comprise" and any other variants are intended to cover the non-exclusive inclusion. For example, a process, method, system, product, or device that includes a series of steps or units is not necessarily limited to those expressly listed steps or units, but may include other steps or units not expressly listed or inherent to such a process, method, product, or device.

According to an aspect of the embodiments of the present disclosure, a method for displaying an ultrasound image is provided.

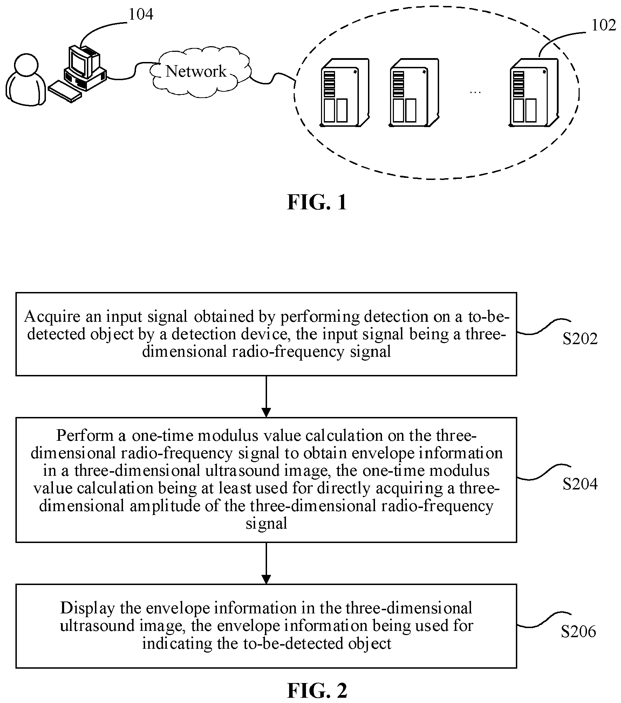

Optionally, in this embodiment, the method for displaying an ultrasound image may be applied to a hardware environment composed of a server 102 and a detection device 104 shown in FIG. 1. As shown in FIG. 1, the server 102 is connected to the detection device 104 by a network. The network includes, but is not limited to, a wide area network, a metropolitan area network or a local area network. The detection device 104 includes an ultrasound device.

The method for displaying an ultrasound image in this embodiment of the present disclosure is performed by the detection device 104 in combination with a display device. The execution process may be described as: acquiring, by the detection device, an input signal obtained by performing detection on a to-be-detected object by the detection device, the input signal being a three-dimensional radio-frequency signal; performing a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; generating, by the detection device, the three-dimensional ultrasound image according to the envelope information and sending, by the detection device, the three-dimensional ultrasound image to the display device; and displaying the envelope information in the three-dimensional ultrasound image on the display device, the envelope information being used for indicating the to-be-detected object.

Optionally, the detection device and the display device may form an integral structure. For example, the detection device 104 shown in FIG. 1 is the integral structure composed of the detection device and the display device. Alternatively, the detection device and the display device may be separate components. The detection device is configured to generate the three-dimensional ultrasound image and the display device is configured to display the three-dimensional ultrasound image.

The following provides a detailed description about the method for displaying an ultrasound image according to an embodiment of the present disclosure.

FIG. 2 is a flowchart of a method for displaying an ultrasound image according to an optional embodiment of the present disclosure. As shown in FIG. 2, the method may include the following steps S202 to S206.

In step S202, an input signal is acquired, the input signal being obtained by performing detection on a to-be-detected object by a detection device, and the input signal being a three-dimensional radio-frequency signal.

In step S204, a one-time modulus value calculation is performed on the three-dimensional radio-frequency signal to obtain envelope information in a three-dimensional ultrasound image, the one-time modulus value calculation being at least used for directly acquiring a three-dimensional amplitude of the three-dimensional radio-frequency signal.

In step S206, the envelope information in the three-dimensional ultrasound image is displayed, the envelope information being used for indicating the to-be-detected object.

Through the foregoing steps S202 to S206, by acquiring the input signal obtained by performing the detection on the to-be-detected object by the detection device, the input signal being the three-dimensional radio-frequency signal; performing the one-time modulus value calculation on the three-dimensional radio-frequency signal to obtain the envelope information in the three-dimensional ultrasound image, the one-time modulus value calculation being at least used for directly acquiring the three-dimensional amplitude of the three-dimensional radio-frequency signal; and displaying the envelope information in the three-dimensional ultrasound image on the display device, the envelope information being used for indicating the to-be-detected object, the to-be-detected object is accurately displayed in the three-dimensional ultrasound image, to achieve a technical effect of improving the accuracy of the three-dimensional ultrasound image, thereby solving the technical problem that the three-dimensional B-mode ultrasound image reconstructed in the related art has a reconstruction error that reduces the accuracy of the three-dimensional B-mode ultrasound image.

In the technical solution provided in step S202, the detection device includes an ultrasound device. The detection device may be configured to detect the to-be-detected object, a type of the to-be-detected object being not specifically limited in the embodiments of the present disclosure. For example, the to-be-detected object may be a human organ (for example, the kidney or the liver). When the detection device detects the to-be-detected object, the detection device may send a detection signal. A signal obtained after the detection signal is reflected by the to-be-detected object is the input signal, where the input signal may be a real signal and the input signal may be a high-frequency three-dimensional radio-frequency signal.

In the technical solution provided in step S204, after the input signal is acquired, in the embodiment of the present disclosure, the one-time modulus value calculation may be performed on the input signal, that is, the one-time modulus value calculation is performed on the three-dimensional radio-frequency signal, to obtain the envelope information in the three-dimensional ultrasound image, where the envelope information in the three-dimensional ultrasound image may be used for indicating the to-be-detected object. The one-time modulus value calculation may at least be used for directly acquiring the three-dimensional amplitude of the three-dimensional radio-frequency signal, where the envelope information may include the three-dimensional amplitude of the three-dimensional radio-frequency signal. In the embodiment of the present disclosure, by performing the one-time modulus value calculation on the three-dimensional radio-frequency signal, the envelope information in the three-dimensional ultrasound image is obtained. Compared with obtaining the three-dimensional ultrasound image by splicing one-dimensional envelope information, the brightness of the to-be-detected object indicated by the envelope information in the three-dimensional ultrasound image according to the embodiment of the present disclosure is greater than the brightness of the to-be-detected object in a one-dimensional ultrasound image or a two-dimensional ultrasound image, to clearly display the to-be-detected object in the three-dimensional ultrasound image, thereby improving the accuracy of the three-dimensional ultrasound image.

The following provides a detailed description about a process of performing the one-time modulus value calculation on the three-dimensional radio-frequency signal to obtain the envelope information in the three-dimensional ultrasound image.

Step S204 of performing the one-time modulus value calculation on the three-dimensional radio-frequency signal includes the following two steps.

In a first step, a first hypercomplex signal corresponding to the three-dimensional radio-frequency signal is acquired, the first hypercomplex signal being a sum of 8 components, and each of the 8 components being represented by modulus values and angles of multiple analytic signals corresponding to the input signal.

In a second step, a modulus value of the first hypercomplex signal is acquired, the modulus value of the first hypercomplex signal being used for representing the three-dimensional amplitude of the three-dimensional radio-frequency signal, and the envelope information including the modulus value of the first hypercomplex signal.

For the above second step, optionally, the acquiring the first hypercomplex signal corresponding to the input signal may include: acquiring a second hypercomplex signal corresponding to the input signal, the second hypercomplex signal including 8 components, and each of the 8 components being represented by a Hilbert transform of the input signal; acquiring a correspondence between the components represented by the Hilbert transform and the modulus values and angles of the multiple analytic signals; and transforming the second hypercomplex signal into the first hypercomplex signal according to the correspondence.

Optionally, the input signal of the three-dimensional radio-frequency signal may be defined as f(x, y, z) herein, and a hypercomplex signal $\psi_{cas}(x, y, z)$ of f(x, y, z) is defined as equation (3):

$$\psi_{cas}(x,y,z) = f(x,y,z) \star \star \star \left\{ \left[\delta(x) + \frac{e_1}{\pi x}\right] \left[\delta(y) + \frac{e_2}{\pi y}\right] \left[\delta(z) + \frac{e_3}{\pi z}\right] \right\}. \quad (3)$$

The hypercomplex signal $\psi_{cas}(x, y, z)$ uses 3 bases of complex units: $e_1$, $e_2$, and $e_3$, to define an imaginary unit. The theoretical foundation thereof is derived from the definition of a biquaternion. The following explains related contents.

When it is defined that $e_1 = e_2 = e_3 = i$, they are a conventional imaginary unit i shown in equation (1).

Conventional one-dimensional envelope detection is implemented by using a one-dimensional analytic signal. For a one-dimensional radio-frequency signal f(x), the one-dimensional Hilbert transform H{f(x)} is calculated, and they are respectively used as a real part and an imaginary part to form a complex signal, that is, the one-dimensional analytic signal $f_A(x)$, as shown in equation (1):

$$f_A(x) = f(x) + iH\{f(x)\}, x \in \mathbb{R} \tag{1},$$

where $i$ is the complex unit and x belongs to a real number R. An amplitude value of the one-dimensional high-frequency signal is shown in equation (2):

$$|f_A(x)| = \sqrt{f(x)^2 + (H\{f(x)\})^2} \tag{2}.$$

If e1, e2, and e3 are different from each other, 8 different imaginary units ($2^3 = 8$) may be generated, as defined by equation (4):

$$[1, i = e_2 e_3, j = e_3 e_1, k = e_1 e_2, \epsilon = -e_1 e_2 e_3, \epsilon i = e_1, \epsilon j = e_2, \epsilon k = e_3] \tag{4},$$

where 1 represents the real part, $\epsilon^2 = 1$, and $e_1{}^2 = e_2{}^2 = e_3{}^2 = -1$.

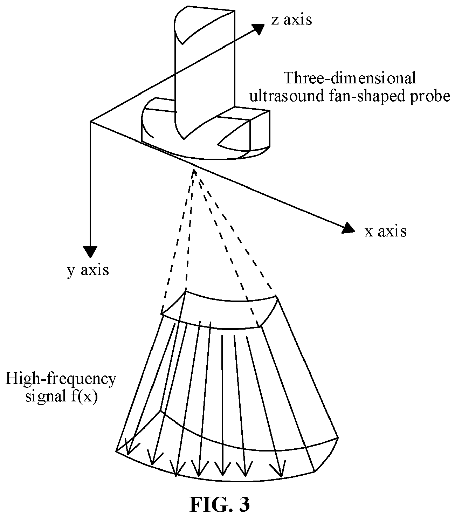

In equation (3), *** represents a 3D convolution calculation. $\delta(x)$, $\delta(y)$, and $\delta(z)$ are Dirac functions. For the three-dimensional radio-frequency signal, the x axis, the y axis and the z axis herein may respectively correspond to physical interpretations of the x axis, the y axis and the z axis in FIG. 3. FIG. 3 is a schematic diagram of sending a radio-frequency signal by a three-dimensional ultrasound fan-shaped probe. The high-frequency signal $f(x)$ represents a one-dimensional radio-frequency signal sent by the ultrasound probe. Multiple one-dimensional radio-frequency signals form one plane and multiple planes form one piece of three-dimensional radio-frequency volume data.

Equation (3) is expended to obtain equation (5):

$$\psi_{cas}(x, y, z) = f(x, y, z) \star \star \star \left\{ \left[\delta(x) + \frac{e_1}{\pi x}\right] \left[\delta(y) + \frac{e_2}{\pi y}\right] \left[\delta(z) + \frac{e_3}{\pi z}\right] \right\}$$

$$= f(x, y, z) \star \star \star \left\{ \delta(x)\delta(y)\delta(z) + \delta(x)\delta(y)\frac{e_3}{\pi z} + \delta(x)\frac{e_2}{\pi y}\delta(z) + \delta(x)\frac{e_2}{\pi y}\frac{e_3}{\pi z} \right.$$

$$\left. + \frac{e_1}{\pi x}\delta(y)\delta(z) + \frac{e_1}{\pi x}\delta(y)\frac{e_3}{\pi z} + \frac{e_1}{\pi x}\frac{e_2}{\pi y}\delta(z) + \frac{e_1}{\pi x}\frac{e_2}{\pi y}\frac{e_3}{\pi z} \right\}, \tag{5}$$

where the convolution calculation in equation (5) is expended, to obtain the following 8 convolution calculations (equations expressed by equation (6)). In addition, according to equation (4), an imaginary unit of each convolution in equation (6) may be calculated, as shown in equation(6):

$$f(x,y,z) \star \star \star [\delta(x)\delta(y)\delta(z)] = f(x,y,z),$$

$$f(x,y,z) \star \star \star [\delta(x)\delta(y)\frac{e_3}{\pi z}] = e_3 H_z\{f\} = \epsilon k H_z\{f\},$$

$$f(x,y,z) \star \star \star [\delta(x)\frac{e_2}{\pi y}\delta(z)] = e_2 H_y\{f\} = \epsilon j H_y\{f\},$$

$$f(x,y,z) \star \star \star [\frac{e_1}{\pi x}\delta(y)\delta(z)] = e_1 H_x\{f\} = \epsilon i H_x\{f\},$$

$$f(x,y,z) \star \star \star [\delta(x)\frac{e_2}{\pi y}\frac{e_3}{\pi z}] = e_2 e_3 H_{yz}\{f\} = i H_{yz}\{f\},$$

$$f(x,y,z) \star \star \star [\frac{e_1}{\pi x}\delta(y)\frac{e_3}{\pi z}] = e_1 e_3 H_{xz}\{f\} = -e_3 e_1 H_{xz}\{f\} = -j H_{xz}\{f\},$$

$$f(x,y,z) \star \star \star [\frac{e_1}{\pi x}\frac{e_2}{\pi y}\delta(z)] = e_1 e_2 H_{xy}\{f\} = k H_{xy}\{f\},$$

$$f(x,y,z) \star \star \star [\frac{e_1}{\pi x}\frac{e_2}{\pi y}\frac{e_3}{\pi z}] = e_1 e_2 e_3 H\{f\} = -\epsilon H\{f\}. \qquad (6)$$

In equation (6), H{f} represents the Hilbert transform of signal $f(x, y, z)$. $H_z${f} represents the Hilbert transform of signal $f(x, y, z)$ in the z direction, $H_y${f} represents the Hilbert transform of signal $f(x, y, z)$ in the y direction, $H_x${f} represents the Hilbert transform of signal $f(x, y, z)$ in the x direction, $H_{yz}${f} represents the Hilbert transform of signal $f(x, y, z)$ in the y direction and the z direction, $H_{xz}${f} represents the Hilbert transform of signal $f(x, y, z)$ in the x direction and the z direction, and $H_{xy}${f} represents the Hilbert transform of signal $f(x, y, z)$ in the x direction and the y direction.

By substituting a result of equation (6) is into equation (5), a hypercomplex signal $\psi_{cas}(x, y, z)$ may be obtained, as shown in equation (7):

$$\psi_{cas}(x,y,z) = f + iH_{yz}\{f\} + j(-H_{xz}\{f\}) + kH_{xy}\{f\}$$
$$+\epsilon(-H\{f\}) + \epsilon i H_x\{f\} + \epsilon j H_y\{f\} + \epsilon k H_z\{f\} \qquad (7)$$

The hypercomplex signal in equation (7) is the second hypercomplex signal in the embodiments of the present disclosure.

In conclusion, the second hypercomplex signal corresponding to the input signal $f(x, y, z)$ of the three-dimensional radio-frequency signal is shown in equation (7). Each component of the hypercomplex signal is represented by the Hilbert transform of the input signal.

Equation (7) is a theoretical value. Next, each component needs to be calculated from the perspective of engineering, and then the amplitude value (theoretical value) of the hypercomplex signal is calculated, thereby acquiring the amplitude value (theoretical value) of the hypercomplex signal from the perspective of engineering.

The foregoing content defines the hypercomplex signal in a form of convolution, and defines the imaginary unit of the hypercomplex signal by using three bases of the biquaternion, which is beneficial since this form of definition is a macroscopic form of a conventional complex number and a quaternion, and may be used for processing three-dimensional data and expressing the conventional complex number (one real part and one imaginary part) and the quaternion (one real part and three imaginary parts) in a downward compatible manner.

A method for indirectly calculating the Hilbert transform in each component of the hypercomplex signal is needed to acquire a mathematical expression of a result of equation (7), that can be implemented in engineering.

Because it is very difficult to directly calculate the Hilbert transform theoretically, a method for indirectly calculating the Hilbert transform is described herein. The method may be implemented in engineering rather than being a theoretical equation.

Engineering implementation means that: it may be implemented by using a common programming language and an open code library.

For the input signal f(x, y, z) of the three-dimensional radio-frequency signal, a calculated single-orthant analytic signal of f(x, y, z) is a signal obtained by performing inverse Fourier transform on a single orthant of a 3D Fourier spectrum of a real signal f(x, y, z). The signal may be calculated by using a Fourier transform function of a general programming language.

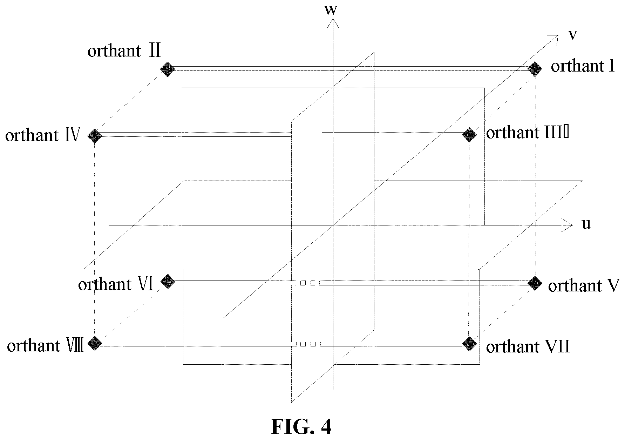

One three-dimensional real signal is first transformed from a 3D real number domain to a 3D frequency domain through the Fourier transform. There are 8 orthants in the 3D frequency domain, as shown in FIG. 4. In the 3D frequency domain, half of the spectrum includes all information about the whole original signal. Therefore, in the 8 orthants of the 3D frequency domain, four adjacent orthants may be selected to include all information about the input signal. FIG. 4 shows the eight orthants in the 3D frequency domain. It can be seen that, orthant I, orthant III, orthant V, and orthant VII are four adjacent orthants.

The eight orthants in the 3D frequency domain are shown in FIG. 4, where u, v, and w are 3 dimensions of the frequency domain.

The following describes a calculation process of four single-orthant analytic signals, as shown in equations (8) to equation (11):

$$
\begin{aligned}
\psi_1(x, y, z) &= f(x, y, z) \star \star \star \left\{ \left[ \delta(x) + \frac{i}{\pi x} \right] \left[ \delta(y) + \frac{i}{\pi y} \right] \left[ \delta(z) + \frac{i}{\pi z} \right] \right\} \\
&= (f - H_{xy}\{f\} - H_{xz}\{f\} - H_{yz}\{f\}) + i (H_x\{f\} + H_y\{f\} + H_z\{f\} - H\{f\}) \\
&= a_1 e^{i\varphi_1} = a_1 \cos\varphi_1 + i a_1 \sin\varphi_1,
\end{aligned} \tag{8}
$$

$$
\begin{aligned}
\psi_3(x, y, z) &= f(x, y, z) \star \star \star \left\{ \left[ \delta(x) + \frac{i}{\pi x} \right] \left[ \delta(y) - \frac{i}{\pi y} \right] \left[ \delta(z) + \frac{i}{\pi z} \right] \right\} \\
&= (f + H_{xy}\{f\} - H_{xz}\{f\} + H_{yz}\{f\}) + i (H_x\{f\} - H_y\{f\} + H_z\{f\} + H\{f\}) \\
&= a_3 e^{i\varphi_3} = a_3 \cos\varphi_3 + i a_3 \sin\varphi_3,
\end{aligned} \tag{9}
$$

$$
\begin{aligned}
\psi_5(x, y, z) &= f(x, y, z) \star \star \star \left\{ \left[ \delta(x) + \frac{i}{\pi x} \right] \left[ \delta(y) + \frac{i}{\pi y} \right] \left[ \delta(z) - \frac{i}{\pi z} \right] \right\} \\
&= (f - H_{xy}\{f\} + H_{xz}\{f\} + H_{yz}\{f\}) + i (H_x\{f\} + H_y\{f\} - H_z\{f\} + H\{f\}) \\
&= a_5 e^{i\varphi_5} = a_5 \cos\varphi_5 + i a_5 \sin\varphi_5,
\end{aligned} \tag{10}
$$

$$
\begin{aligned}
\psi_7(x, y, z) &= f(x, y, z) \star \star \star \left\{ \left[ \delta(x) + \frac{i}{\pi x} \right] \left[ \delta(y) - \frac{i}{\pi y} \right] \left[ \delta(z) - \frac{i}{\pi z} \right] \right\} \\
&= (f + H_{xy}\{f\} + H_{xz}\{f\} - H_{yz}\{f\}) + i (H_x\{f\} - H_y\{f\} - H_z\{f\} - H\{f\}) \\
&= a_7 e^{i\varphi_7} = a_7 \cos\varphi_7 + i a_7 \sin\varphi_7.
\end{aligned} \tag{11}
$$

$\psi_1(x, y, z)$, $\psi_3(x, y, z)$, $\psi_5(x, y, z)$, and $\psi_7(x, y, z)$, represent single-orthant analytic signals respectively acquired from orthant I, orthant III, orthant V, and orthant VII of the frequency domain in FIG. 4. The difference between the signal and the definition in equation (3) is that: the definition uses the unique imaginary unit i, that is the most conventional definition of the complex number (a complex number including one real part and one imaginary part). Similar to the calculation method of equation (6), a three-dimensional convolution of equations (8) to (11) has 8 components. The 8 components may each be represented by the Hilbert transform of the input signal f(x, y, z), which form real parts and imaginary parts of the single-orthant analytic signals.

Further, the foregoing equations also define modulus values and angles of the single-orthant analytic signals (that is, a form of polar coordinates of a complex number $\psi_1(x, y, z)$). For example, in equation (8), $\alpha_1(x, y, z)$, represents the modulus value in the form of the polar coordinates of the complex number $\psi_1(x, y, z)$, ($\alpha_1$ herein may also be referred to as an amplitude value). In equation (8), $\alpha_1(x, y, z)$ is shortened to $\alpha_1$. $\varphi_1(x, y, z)$ represents the angle in the form of the polar coordinates of the complex number $\psi_1(x, y, z)$, ($\varphi_1$ may also be referred to as the phase herein). Similarly, in equation (8), $\varphi_1(x, y, z)$, is shortened to $\varphi_1$. $\alpha_1$ and $\varphi_1$ may be calculated according to equation (12):

$$a_1 = \sqrt{(f - H_{xy}\{f\} - H_{xz}\{f\} - H_{yz}\{f\})^2 + (H_x\{f\} + H_y\{f\} + H_z\{f\} - H\{f\})^2}$$

$$\varphi_1 = arctan\left(\frac{H_x\{f\} + H_y\{f\} + H_z\{f\} - H\{f\}}{f - H_{xy}\{f\} - H_{xz}\{f\} - H_{yz}\{f\}}\right) \qquad (12)$$

The relationship between the modulus value $\alpha_1$, the angle $\varphi_1$ and the Hilbert transform may be obtained by equation (8), as shown in equation (13):

$$a_1 \cos\varphi_1 = f - H_{xy}\{f\} - H_{xz}\{f\} - H_{yz}\{f\}$$
$$a_1 \sin\varphi_1 = H_x\{f\} + H_y\{f\} + H_z\{f\} - H\{f\} \qquad (13)$$

Similarly, the correspondence between the modulus values, the angles and the Hilbert transform of the other three single-orthant analytic signals may be obtained from equations (9) to (11), as shown in equation (14):

$$f = \tfrac{1}{4}\left(a_1 \cos\varphi_1 + a_3 \cos\varphi_3 + a_5 \cos\varphi_5 + a_7 \cos\varphi_7\right),$$
$$H_{yz}\{f\} = \tfrac{1}{4}\left(-a_1 \cos\varphi_1 + a_3 \cos\varphi_3 + a_5 \cos\varphi_5 - a_7 \cos\varphi_7\right),$$
$$-H_{xz}\{f\} = \tfrac{1}{4}\left(a_1 \cos\varphi_1 + a_3 \cos\varphi_3 - a_5 \cos\varphi_5 - a_7 \cos\varphi_7\right),$$
$$H_{xy}\{f\} = \tfrac{1}{4}\left(-a_1 \cos\varphi_1 + a_3 \cos\varphi_3 - a_5 \cos\varphi_5 + a_7 \cos\varphi_7\right),$$
$$-H\{f\} = \tfrac{1}{4}\left(a_1 \sin\varphi_1 - a_3 \sin\varphi_3 - a_5 \sin\varphi_5 + a_7 \sin\varphi_7\right),$$
$$H_x\{f\} = \tfrac{1}{4}\left(a_1 \sin\varphi_1 + a_3 \sin\varphi_3 + a_5 \sin\varphi_5 + a_7 \sin\varphi_7\right),$$
$$H_y\{f\} = \tfrac{1}{4}\left(a_1 \sin\varphi_1 - a_3 \sin\varphi_3 + a_5 \sin\varphi_5 - a_7 \sin\varphi_7\right),$$
$$H_z\{f\} = \tfrac{1}{4}\left(a_1 \sin\varphi_1 + a_3 \sin\varphi_3 - a_5 \sin\varphi_5 - a_7 \sin\varphi_7\right). \qquad (14)$$

Equation (14) may be used for representing a correspondence between the components represented by the Hilbert transform and the modulus value and angle of the analytic signal in the embodiments of the present disclosure.
Equation (14) uses the modulus value and angle of the analytic signal of the input signal to represent the Hilbert transform. It is relatively difficult to obtain, through a calculation, the left part of equation (14) in engineering, while the right part of equation (14) may be obtained through a calculation of a library function of the Fourier transform of the conventional programming language.
By substituting the result of equation (14) into equation (7), the hypercomplex signal $\psi_{cas}(x, y, z)$ defined in mathematical theory is calculated by the library function of the Fourier transform of the conventional programming language, which is the expression shown in equation (15):

$$\psi_{cas} = \frac{1}{4}\Big[\; \left(a_1 \cos\varphi_1 + a_3 \cos\varphi_3 + a_5 \cos\varphi_5 + a_7 \cos\varphi_7\right) \qquad (15)$$
$$+ i\left(-a_1 \cos\varphi_1 + a_3 \cos\varphi_3 + a_5 \cos\varphi_5 - a_7 \cos\varphi_7\right)$$
$$+ j\left(a_1 \cos\varphi_1 + a_3 \cos\varphi_3 - a_5 \cos\varphi_5 - a_7 \cos\varphi_7\right)$$
$$+ k\left(-a_1 \cos\varphi_1 + a_3 \cos\varphi_3 - a_5 \cos\varphi_5 + a_7 \cos\varphi_7\right)$$
$$+ \epsilon\left(a_1 \sin\varphi_1 - a_3 \sin\varphi_3 - a_5 \sin\varphi_5 + a_7 \sin\varphi_7\right)$$
$$+ \epsilon i\left(a_1 \sin\varphi_1 + a_3 \sin\varphi_3 + a_5 \sin\varphi_5 + a_7 \sin\varphi_7\right)$$
$$+ \epsilon j\left(a_1 \sin\varphi_1 - a_3 \sin\varphi_3 + a_5 \sin\varphi_5 - a_7 \sin\varphi_7\right)$$
$$+ \epsilon k\left(a_1 \sin\varphi_1 + a_3 \sin\varphi_3 - a_5 \sin\varphi_5 - a_7 \sin\varphi_7\right)\Big].$$

The hypercomplex signal in equation (15) is the first hypercomplex signal in this embodiment of the present disclosure.

In conclusion, in the foregoing description, the content of the hypercomplex signal $\psi_{cas}(x, y, z)$ is theoretically converted into an expression in another form, in order to acquire an expression of the hypercomplex signal $\psi_{cas}(x, y, z)$ that can be implemented in engineering, as shown in equation (15).

After acquiring the first hypercomplex signal shown in equation (15), the modulus value of the first hypercomplex signal may be calculated. The process of calculating the modulus value $|\psi_{cas}(x, y, z)|$ of the hypercomplex signal may be described as follows.

Following two properties of biquaternions are to be used when the modulus value is calculated.

Biquaternion property 1: multiplication of the biquaternion.

A biquaternion A may be expressed by equation (16):

$$
\begin{aligned}
A &= p + \epsilon q \\
&= (p_0 + ip_1 + jp_2 + kp_3) + \epsilon(q_0 + iq_1 + jq_2 + kq_3) \\
&= p_0 + ip_1 + jp_2 + kp_3 + \epsilon q_0 + \epsilon iq_1 + \epsilon jq_2 + \epsilon kq_3
\end{aligned}
\tag{16}
$$

Both p and q are quaternions. Another biquaternion is defined as $B = p' + \varepsilon q'$, and the product of the two biquaternions is shown in equation (17):

$$
AB = (p + \epsilon q)(p' + \epsilon q') = (pp' + qq') + \epsilon(pq' + qp')
\tag{17}
$$

where the quaternion product theory of the quaternions $p, q, p'$, and $q'$ is not described herein.

Biquaternion property 2: conjugate of the biquaternion.

Conjugate of the biquaternion A may be defined as $Ac$, as shown in equation (18):

$$
\begin{aligned}
A_c &= p_c + \epsilon q_c \\
&= (p_0 - ip_1 - jp_2 - kp_3) + \epsilon(q_0 - iq_1 - jq_2 - kq_3) \\
&= p_0 - ip_1 - jp_2 - kp_3 + \epsilon q_0 - \epsilon iq_1 - \epsilon jq_2 - \epsilon kq_3
\end{aligned}
\tag{18}
$$

where $p_c$ is conjugate of quaternion p.

In order to calculate the modulus value $|\psi_{cas}(x, y, z)|$ of the hypercomplex signal, equations (16) to (19) are to be first used to calculate the product of $\psi_{cas}(x, y, z)$ and the conjugate thereof, that is, $\psi_{cas}(\psi_{cas})_c$, as shown in equation (19):

$$
\begin{aligned}
\psi_{cas}(\psi_{cas})_c &= \frac{a_1^2 + a_3^2 + a_5^2 + a_7^2}{4} + i(0) + j(0) + k(0) \\
&\quad + \epsilon \frac{a_1 a_3 \sin(\varphi_1 - \varphi_3) - a_5 a_7 \sin(\varphi_5 - \varphi_7)}{2} + \epsilon i(0) + \epsilon j(0) + \epsilon k(0) \\
&= \frac{a_1^2 + a_3^2 + a_5^2 + a_7^2}{4} + \epsilon \frac{a_1 a_3 \sin(\varphi_1 - \varphi_3) - a_5 a_7 \sin(\varphi_5 - \varphi_7)}{2},
\end{aligned}
\tag{19}
$$

as can be seen from equation (19) that, a result of $\psi_{cas}(\psi_{cas})_c$, only includes two parts, where one part is a real part, that is,

$$
\frac{a_1^2 + a_3^2 + a_5^2 + a_7^2}{4},
$$

the other part takes $\varepsilon$ as an imaginary unit, that is,

$$\epsilon \frac{a_1 a_3 \sin(\varphi_1 - \varphi_3) - a_5 a_7 \sin(\varphi_5 - \varphi_7)}{2},$$

which is referred to as a "pseudo real" part of a biquaternion. Other imaginary parts are all 0. The result is greatly helpful in calculating the modulus value $|\psi_{cas}(x, y, z)|$. The following describes a process of calculating the modulus value $|\psi_{cas}(x, y, z)|$.

First, a polar coordinate form of a hypercomplex signal $\psi_{cas}(x, y, z)$ is defined as:

$$\psi_{cas} = |\psi_{cas}| e^{\epsilon \phi} a,$$

where $|\psi_{cas}|$ is a modulus value, $a$ is a unit biquaternion (that has a property: a product of conjugate and itself is 1, namely, $a(a_c) = 1$), and $\phi$ is an angle of the biquaternion. As shown in equation (20):

$$\psi_{cas}(\psi_{cas})_c = |\psi_{cas}|^2 e^{2\epsilon \phi} = |\psi_{cas}|^2 [ch(2\phi) + \epsilon sh(2\phi)], \qquad (20)$$

where $ch()$ and $sh()$ are a hyperbolic cosine function and a hyperbolic sine function respectively. The derivation process of the equation is shown as follows:

$$\begin{aligned}
e^{2\epsilon \phi} &= 1 + 2\epsilon\phi + \frac{(2\epsilon\phi)^2}{2!} + \frac{(2\epsilon\phi)^3}{3!} + \ldots + \frac{(2\epsilon\phi)^{2r}}{(2r)!} + +\frac{(2\epsilon\phi)^{2r+1}}{(2r+1)!} + \ldots \\
&= \left[ 1 + \frac{(2\epsilon\phi)^2}{2!} + \frac{(2\epsilon\phi)^4}{4!} + \ldots + \frac{(2\epsilon\phi)^{2r}}{(2r)!} + \ldots \right] \\
&\quad + \left[ 2\epsilon\phi + \frac{(2\epsilon\phi)^3}{3!} + \frac{(2\epsilon\phi)^5}{5!} + \ldots + \frac{(2\epsilon\phi)^{2r+1}}{(2r+1)!} + \ldots \right] \\
&= \left[ 1 + \frac{(2\epsilon\phi)^2}{2!} + \frac{(2\epsilon\phi)^4}{4!} + \ldots + \frac{(2\epsilon\phi)^{2r}}{(2r)!} + \ldots \right] \\
&\quad + \epsilon \left[ 2\phi + \frac{(2\phi)^3}{3!} + \frac{(2\phi)^5}{5!} + \ldots + \frac{(2\phi)^{2r+1}}{(2r+1)!} + \ldots \right] \\
&= ch(2\phi) + \epsilon sh(2\phi),
\end{aligned}$$

where r represents a positive complex number.

In order to simplify the calculation, two symbols $M$ and $N$ may be used to represent equation (20):

$$\psi_{cas}(\psi_{cas})_c = M + \epsilon N,$$

where $M$ represents real parts in equation (19) and equation (20), and $N$ represents "pseudo real" parts in equation (19) and equation (20). The following may be obtained:

$$M^2 - N^2 = |\psi_{cas}|^4 [ch(2\phi)^2 - sh(2\phi)^2] = |\psi_{cas}|^4.$$

Therefore, $|\psi_{cas}| = (M^2 - N^2)^{1/4}$, and by substituting symbols $M$ and $N$ into equation (19), equation (21) is obtained:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

where a result of equation (21) is the modulus value $|\psi_{cas}(x, y, z)|$ of the first hypercomplex signal. Elements representing the modulus value come from calculations of equation (8) to equation (11), which can be implemented in engineering. Input information is modulus values $\alpha_1(x, y, z)$, $\alpha_3(x, y, z)$, $\alpha_5(x, y, z)$, and $\alpha_7(x, y, z)$, and angles $\varphi_1(x, y, z)$, $\varphi_3(x, y, z)$, $\varphi_5(x, y, z)$, and $\varphi_7(x, y, z)$, of polar coordinates of equations (8) to (11). Output is the modulus value of the first hypercomplex signal, that is, an envelope signal $|\psi_{cas}(x, y, z)|$.

That is to say, in the embodiments of the present disclosure, the modulus value of the first hypercomplex signal is acquired according to the following equation (21):

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

$$(21)$$

where $|\psi_{cas}|$ represents the modulus value of the first hypercomplex signal, $\alpha_1$ is a modulus value of a first analytic signal, $\varphi_1$ is an angle of the first analytic signal, the first analytic signal is an analytic signal that is in the first orthant of 8 orthants in a three-dimensional frequency domain and that corresponds to the input signal, $\alpha_3$, is a modulus value of a third analytic signal, $\varphi_3$ is an angle of the third analytic signal, the third analytic signal is an analytic signal that is in the third orthant of the 8 orthants in the three-dimensional frequency domain and that corresponds to the input signal, $\alpha_5$ is a modulus value of a fifth analytic signal, $\varphi_5$ is an angle of the fifth analytic signal, the fifth analytic signal is an analytic signal that is in the fifth orthant of the 8 orthants in the three-dimensional frequency domain and that corresponds to the input signal, $\alpha_7$ is a modulus value of a seventh analytic signal, $\varphi_7$ is an angle of the seventh analytic signal, the seventh analytic signal is an analytic signal that is in the seventh orthant of the 8 orthants in the three-dimensional frequency domain and that corresponds to the input signal, and the multiple analytic signals include the first analytic signal, the third analytic signal, the fifth analytic signal, and the seventh analytic signal.

After acquiring the modulus value of the first hypercomplex signal shown in equation (21), the envelope information used for indicating the to-be-detected object in the three-dimensional ultrasound image is obtained. In the embodiments of the present disclosure, the three-dimensional ultrasound image is generated according to the envelope information. A process of generating the three-dimensional ultrasound image according to the envelope information is not specifically limited herein, and may include, but is not limited to, image processing means such as denoising.

In the technical solution provided in step S206, after generating the three-dimensional ultrasound image, the envelope information in the three-dimensional ultrasound image is displayed on the display device. The display device and the detection device may form an integral structure, or the display device and the detection device may be separate components. In a case that the display device and the detection device are independent of each other, after the detection device generates the three-dimensional ultrasound image, the three-dimensional ultrasound image may be sent to the display device for displaying, so that a user may observe the to-be-detected object from the display device clearly and intuitively.

Using the method for displaying an ultrasound image in this embodiment of the present disclosure, because the envelope information used for indicating the to-be-detected object in the three-dimensional ultrasound image is obtained by performing a one-time modulus value calculation on the three-dimensional radio-frequency signal, rather than by splicing the one-dimensional envelope information, the brightness and definition of the to-be-detected object indicated by the envelope information in the three-dimensional ultrasound image are greater than the brightness and definition of the to-be-detected object in a one-dimensional ultrasound image or a two-dimensional ultrasound image. Therefore, with the embodiments of the present disclosure, the to-be-detected object be more clearly displayed in the three-dimensional ultrasound image, thereby improving the accuracy of the three-dimensional ultrasound image.

The method for displaying an ultrasound image provided in the present disclosure may be used to directly obtain the three-dimensional envelope in the B-mode ultrasound imaging. In the present disclosure, based on a form of 3D convolution and a form of Clifford algebra biquaternion, an analytic signal of high-dimensional hypercomplex numbers is defined to calculate the three-dimensional amplitude of the three-dimensional radio-frequency signal at a time, that is, the three-dimensional ultrasound image of the three-dimensional radio-frequency signal. The Hilbert transform is used to implement an engineering implementation for the provided hypercomplex signal and its modulus value. Compared with a conventional method for reconstructing a B-mode ultrasound image by splicing one-dimensional envelope signals of one-dimensional ultrasound radio-frequency signals according to spatial positions, the present disclosure completely abandons the method for obtaining a B-mode ultrasound image by splicing one-dimensional envelope signals, to avoid a reconstruction error of the three-dimensional B-mode ultrasound image that is formed by splicing the one-dimensional envelope signals. In addition, the present disclosure is further applicable to one-time envelope detection of a two-dimensional B-mode ultrasound image and a three-dimensional B-mode ultrasound image.

The present disclosure may be applied to a device that performs an envelope calculation on the three-dimensional radio-frequency signal, for example, applied to B-mode imaging of a three-dimensional ultrasound device. As shown in FIG. 3, in the three-dimensional B-mode image acquired by using a three-dimensional fan-shaped probe, the method may be used for implementing one-time imaging on the three-dimensional data.

The present disclosure implements the one-time modulus value calculation on a three-dimensional radio-frequency ultrasound signal, to acquire a three-dimensional envelope image thereof (that is, the image indicated by the envelope information in the foregoing embodiment of the present disclosure). The modulus value of the three-dimensional radio-frequency ultrasound signal herein refers to the three-dimensional envelope image. On the basis of the three-dimensional envelope image, three-dimensional ultrasound image may be obtained by using any two-dimensional or three-dimensional image post-processing algorithm.

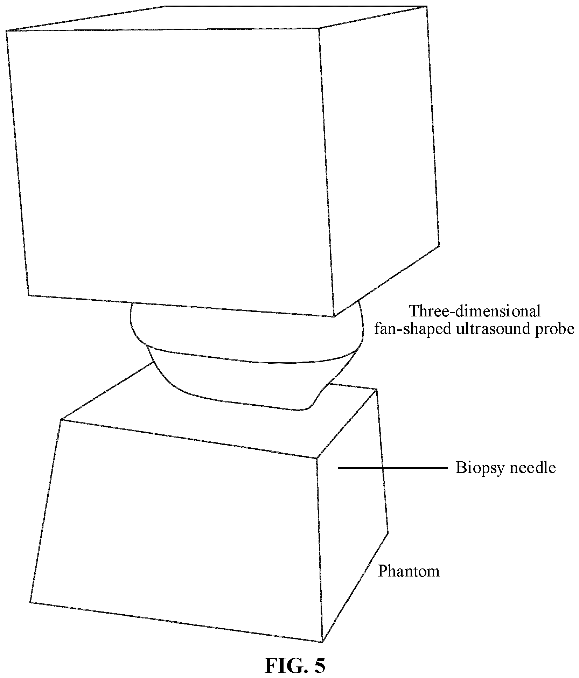

As shown in FIG. 5, a platform where a 3D fan-shaped ultrasound probe is used to acquire a three-dimensional radio-frequency signal is described by using an example. A phantom is under the three-dimensional fan-shaped ultrasound probe to imitate a human body. A biopsy needle is inserted into the phantom to imitate an experiment of inserting the biopsy needle into the human body to acquire human tissue for a subsequent biopsy. In a process of acquiring the human tissue by the biopsy needle, a doctor uses the ultrasound probe to observe a location of the biopsy needle in the human body, aiming to make a needle of the probe reach a predetermined tissue location.

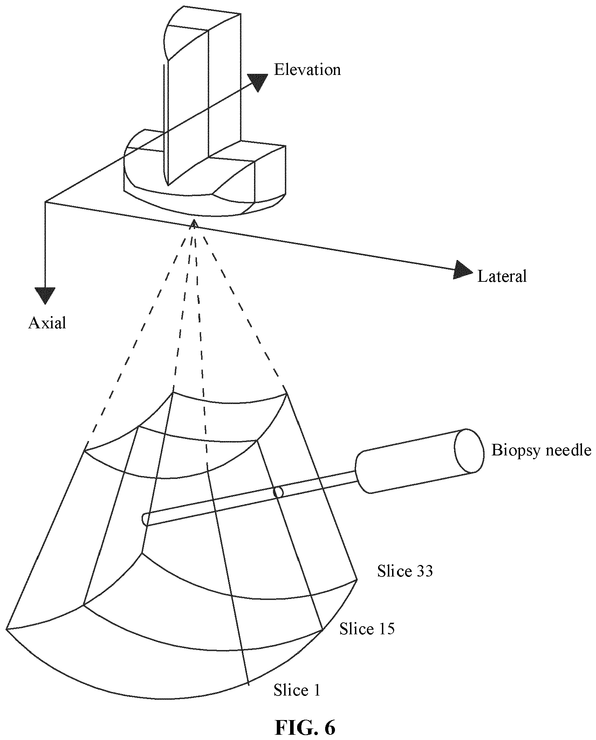

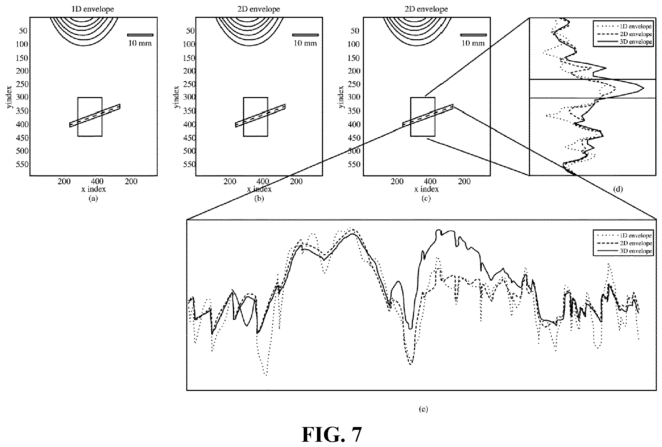

FIG. 6 is marked with 3 terms of coordinate axis directions used in the three-dimensional ultrasound: axial, lateral, and elevation. In FIG. 6, the x axis and the y axis of the three-dimensional radio-frequency signal form a slice. The z axis is an elevation axis that represents different two-dimensional slices. The ultrasound envelope image of the three-dimensional radio-frequency signal acquired in FIG. 6 is calculated separately by using the existing methods and the method provided in the present disclosure. FIG. 7 shows a part of the result, which is a result of slice 15 in FIG. 6. The location of the biopsy needle is on the slice. FIG. 7a and FIG. 7b are results calculated by using the existing one-dimensional method and two-dimensional method. FIG. 7c is a result calculated by using the method according to the present disclosure. In FIG. 7c, a white area that is highlighted, diagonal, long and thin is the location of the biopsy needle. The higher brightness indicates that the biopsy needle is more apparently displayed on the ultrasound envelope image. In order to compare details, in FIG. 7d, profiles in a vertical direction of the image are acquired. FIG. 7e shows profiles along the direction of the probe. Higher values of the profiles indicate that the brightness is higher, that is, the probe is more clearly displayed on the envelope image. It can be seen from the values of the profiles that, the brightness in most of the location of the probe obtained by using the three-dimensional method provided in the present disclosure is higher than those obtained by using the one-dimensional method and the two-dimensional method.

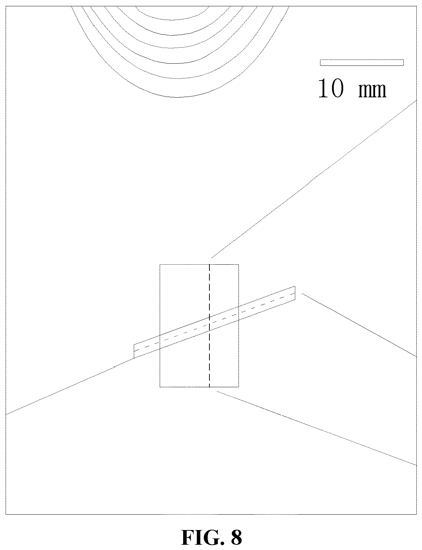

FIG. 8 is enlarged FIG. 7c. In FIG. 8, a diagonal quadrangular box is the location of the biopsy needle. Two imaginary lines are locations of the profiles in FIG. 7d and FIG. 7e.

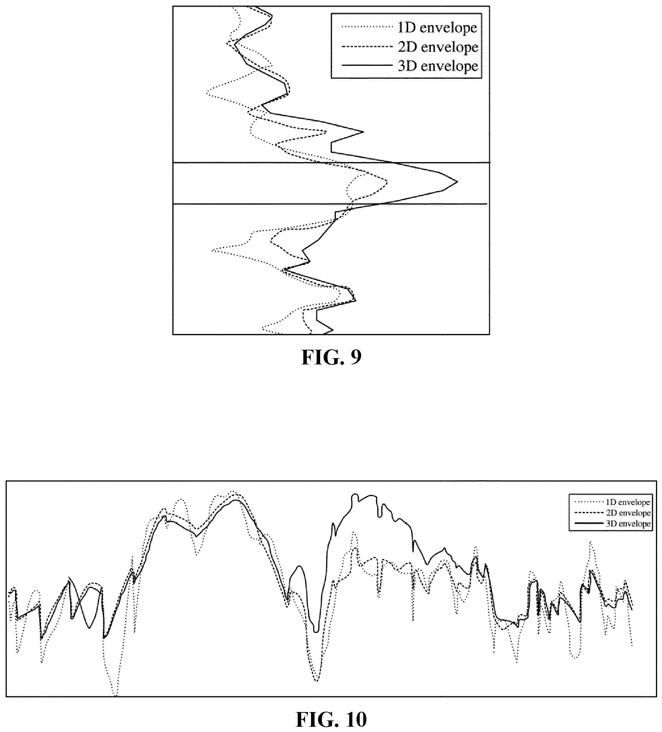

FIG. 9 is enlarged FIG. 7d. FIG. 10 is enlarged FIG. 7e. The middle of two horizontal lines in FIG. 9 is the location of the biopsy needle. The foregoing examples are two results based on the three-dimensional radio-frequency data of the fan-shaped ultrasound probe.

In order to prove the universality of the present disclosure, the following example is a calculation of the three-dimensional envelope image based on the three-dimensional radio-frequency data of the linear probe. A similar conclusion may also be drawn: the three-dimensional envelope image of the solution according to the present disclosure can better display location information of the needle.

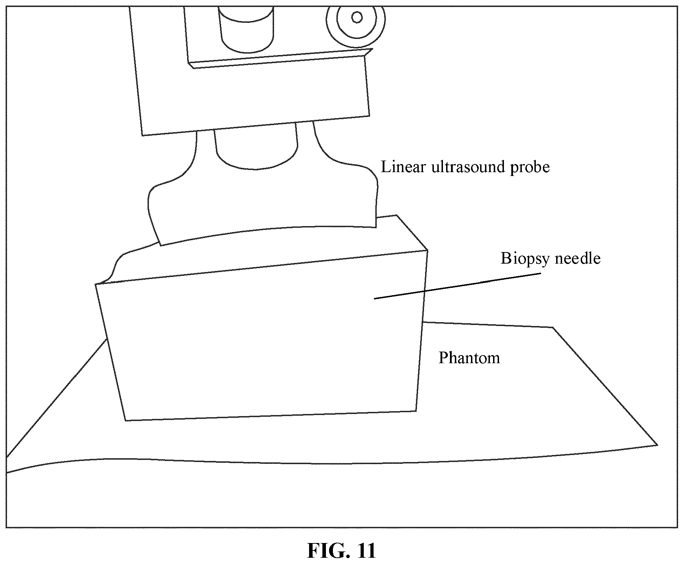

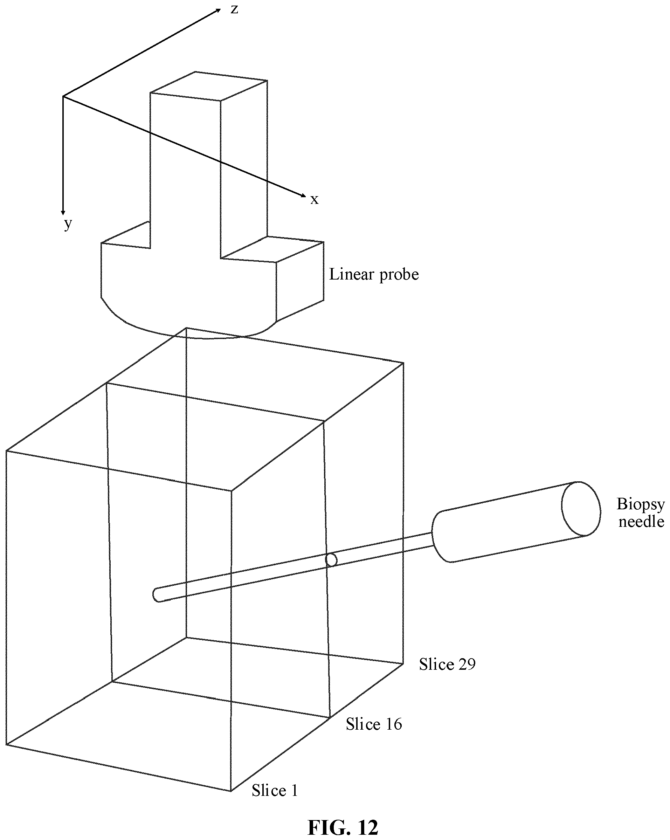

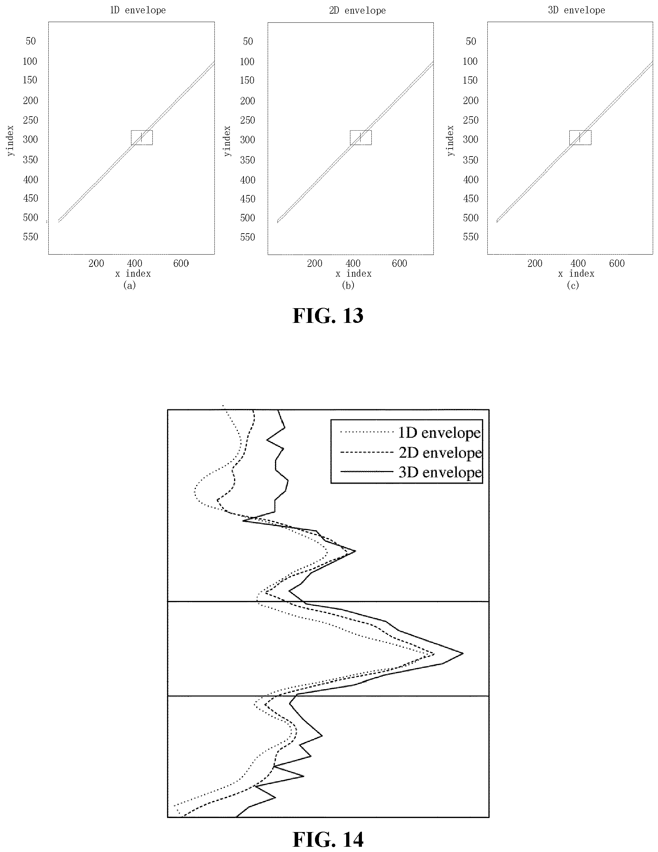

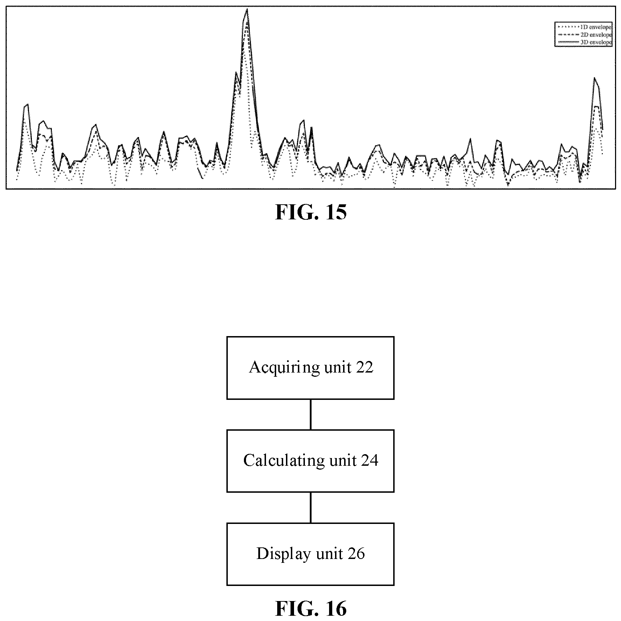

FIG. 11 is a schematic diagram of a linear ultrasound probe. FIG. 12 is a coordinate axis example of data acquired by the linear ultrasound probe, and the data is in a cubic structure. FIG. 13 shows results of a three-dimensional envelope of the linear ultrasound probe, FIG. 13a shows a result of the one-dimensional method, FIG. 13b shows a result of the two-dimensional method, and FIG. 13c shows a result of the method of the present disclosure. FIG. 14 shows a brightness comparison of pixels of the profiles in a vertical direction in the quadrangle. The portion between the two horizontal lines is the location of the biopsy needle. The brightness of the result of the three-dimensional method is the highest. The needle is displayed most apparently. FIG. 15 shows a brightness comparison of pixels of the profiles along the direction of the needle in the quadrangle. The whole curve is the location of the biopsy needle in the image. The brightness of the result of the three-dimensional method is the highest, and the needle is displayed most apparently.

The solution of the present disclosure may solve envelope calculation of all kinds of three-dimensional high-frequency signals mathematically. At an application level, the solution is applicable to the three-dimensional signal that is a high-frequency signal in one dimension or two dimensions but not a high-frequency signal in other dimensions. Therefore, the solution can be potentially applied to various physics and engineering application problems related to a modulus value calculation of three-dimensional high-frequency signals, such as, high-frequency signal communications, high-frequency radar signal demodulation, encryption of images by using high-frequency information, and decryption requiring a calculation of envelope information of signals.

To make the description simple, the foregoing method embodiments are stated as a series of action combinations. However, a person skilled in the art needs to know that the present disclosure is not limited on the described sequence of the actions because according to the present disclosure, certain steps may use another sequence or may be simultaneously performed. In addition, it is to be understood by a person skilled in the art that the embodiments described in the specification all belong to exemplary embodiments and the actions and modules are not mandatory to the present disclosure.

According to the foregoing descriptions of implementations, a person skilled in the art may clearly learn that the method according to the foregoing embodiments may be implemented by using software and a necessary general hardware platform, or certainly may be implemented by using hardware. However, in most cases, the former is a better implementation. Based on such an understanding, the technical solutions in the present disclosure essentially or the part contributing on the related art may be implemented in the form of a software product. The computer software product is stored in a storage medium (such as a read-only memory (ROM)/random access memory (RAM), a magnetic disk, or an optical disc), and includes several instructions for instructing a terminal device (which may be a mobile phone, a computer, a server, a network device, and the like) to perform the method described in the embodiments of the present disclosure.

According to another aspect of the embodiments of the present disclosure, an apparatus for displaying an ultrasound image for implementing the method for displaying an ultrasound image is further provided. FIG. 16 is a schematic diagram of an apparatus for displaying an ultrasound image according to an optional embodiment of the present disclosure. As shown in FIG. 16, the apparatus may include:

an acquiring unit 22, configured to acquire an input signal obtained by performing detection on a to-be-detected object by a detection device, the input signal being a three-dimensional radio-frequency signal; a calculating unit 24, configured to perform a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and a display unit 26, configured to display the envelope information in the three-dimensional ultrasound image on a display device, the envelope information being used for indicating the to-be-detected object.

The acquiring unit 22 in this embodiment may be configured to perform step S202 in the embodiments of the present disclosure, the calculating unit 24 in this embodiment may be configured to perform step S204 in the embodiments of the present disclosure, and the display unit 26 in this embodiment may be configured to perform step S206 in the embodiments of the present disclosure.

Implemented examples and application scenarios of the foregoing modules are the same as those of the corresponding steps, but are not limited to the content disclosed in the foregoing embodiments. The foregoing modules may be run in the hardware environment shown in FIG. 1 as a part of the apparatus, and may be implemented by software, or may be implemented by hardware.

The calculating unit 24 includes a first acquiring module, configured to acquire a first hypercomplex signal corresponding to the three-dimensional radio-frequency signal, the first hypercomplex signal being a sum of 8 components, and each of the 8 components being represented by modulus values and angles of multiple analytic signals corresponding to the input signal; and a second acquiring module, configured to acquire a modulus value of the first hypercomplex signal, the modulus value of the first hypercomplex signal being used for representing the three-dimensional amplitude of the three-dimensional radio-frequency signal, and envelope information including the modulus value of the first hypercomplex signal.

Optionally, the first acquiring module may include: a first acquiring submodule, configured to acquire a second hypercomplex signal corresponding to the three-dimensional radio-frequency signal, the second hypercomplex signal including 8 components, and each of the 8 components being represented by the Hilbert transform of the input signal; a second acquiring submodule, configured to acquire a correspondence between the components represented by the Hilbert transform and the modulus values and angles of the multiple analytic signals; and a transforming module, configured to transform the second hypercomplex signal into the first hypercomplex signal according to the correspondence.

The second acquiring module is configured to acquire the modulus value of the first hypercomplex signal according to the following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

where $|\psi_{cas}|$ represents the modulus value of the first hypercomplex signal, $\alpha_1$ is a modulus value of a first analytic signal, $\varphi_1$ is an angle of the first analytic signal, the first analytic signal is an analytic signal that is in the first orthant of 8 orthants in a three-dimensional frequency domain and that corresponds to the input signal, $\alpha_3$ is a modulus value of a third analytic signal, $\varphi_3$ is an angle of the third analytic signal, the third analytic signal is an analytic signal that is in the third orthant of the 8 orthants in the three-dimensional frequency domain and that corresponds to the input signal, $\alpha_5$ is a modulus value of a fifth analytic signal, $\varphi_5$ is an angle of the fifth analytic signal, the fifth analytic signal is an analytic signal that is in the fifth orthant of the 8 orthants in the three-dimensional frequency domain and that corresponds to the input signal, $\alpha_7$ is a modulus value of a seventh analytic signal, $\varphi_7$ is an angle of the seventh analytic signal, the seventh analytic signal is an analytic signal that is in the seventh orthant of the 8 orthants in the three-dimensional frequency domain and that corresponds to the input signal, and the multiple analytic signals include the first analytic signal, the third analytic signal, the fifth analytic signal, and the seventh analytic signal.

The brightness of the to-be-detected object indicated by the envelope information in the three-dimensional ultrasound image is greater than the brightness of the to-be-detected object in a one-dimensional ultrasound image or a two-dimensional ultrasound image.

Implemented examples and application scenarios of the foregoing modules are the same as those of the corresponding steps, but are not limited to the content disclosed in the foregoing embodiments. The foregoing modules may be run in the hardware environment shown in FIG. 1 as a part of the apparatus, and may be implemented by software, or may be implemented by hardware.

Through the foregoing modules, the to-be-detected object is accurately displayed in the three-dimensional ultrasound image, to achieve a technical effect of improving the accuracy of the three-dimensional ultrasound image, thereby solving the technical problem that the three-dimensional B-mode ultrasound image reconstructed in the related art has a reconstruction error that reduces the accuracy of the three-dimensional B-mode ultrasound image.

According to still another aspect of the embodiments of the present disclosure, an electronic device for implementing the method for displaying an ultrasound image is further provided.

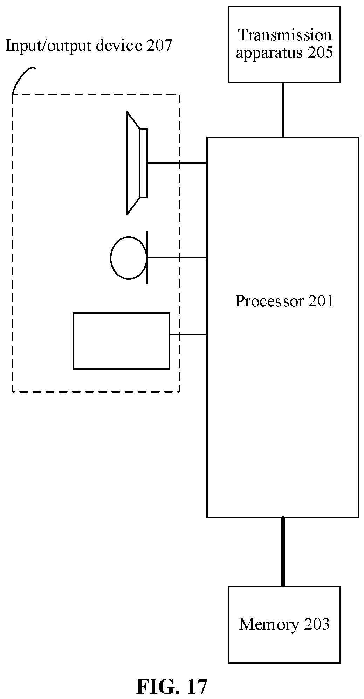

FIG. 17 is a structural block diagram of an electronic device according to an embodiment of the present disclosure. As shown in FIG. 17, the electronic device may include: one or more (only one processor is shown in the figure) processors 201 and a memory 203, the memory 203 storing a computer program, and the processor 201 being configured to run the computer program to perform the method for displaying an ultrasound image according to the embodiments of the present disclosure.

The memory 203 may be configured to store a computer program and a module, for example, a program instruction/module corresponding to the method and apparatus for displaying an ultrasound image in the embodiments of the present disclosure, and the processor 201 performs various functional applications and data processing by running the computer program and the module stored in the memory 203, that is, implementing the foregoing method for displaying an ultrasound image. The memory 203 may include a high-speed random access memory, and may further include a non-volatile memory, for example, one or more magnetic storage apparatuses, flash memories, or other non-volatile solid-state memories. In some embodiments, the memory 203 may further include memories that are remotely disposed relative to the processor 201, and the remote memories may be connected to a terminal via a network. Examples of the network include, but are not limited to, the Internet, an intranet, a local area network, a mobile communications network, and a combination thereof.

Optionally, as shown in FIG. 17, the electronic device may further include: a transmission apparatus 205 and an input/output device 207. The transmission apparatus 205 is configured to receive or send data through a network. Specific instances of the foregoing network may include a wired network and a wireless network. In an example, the transmission apparatus 205 includes a network interface controller (NIC), and the network interface controller may be connected to another network device or a router by using a network cable, so as to communicate with the Internet or a local area network. In an example, the transmission apparatus 205 is a radio frequency (RF) module, and the radio frequency module is configured to communicate with the Internet in a wireless manner.

A person of ordinary skill in the art may understand that, the structure shown in FIG. 17 is only schematic. The electronic device may be a terminal device such as a smartphone (such as an Android mobile phone or an iOS mobile phone), a tablet computer, a palmtop computer, a mobile Internet device (MID), or a PAD. FIG. 17 does not constitute a limitation on a structure of the foregoing electronic device. For example, the electronic device may further include more or fewer components (for example, a network interface and a display apparatus) than those shown in FIG. 17, or has a configuration different from that shown in FIG. 17.

Optionally, in this embodiment, the memory 203 may be configured to store the computer program.

Optionally, in this embodiment, the processor is configured to run the computer program for performing the following steps: acquiring an input signal obtained by performing detection on a to-be-detected object by a detection device, the input signal being a three-dimensional radio-frequency signal; performing a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and displaying the envelope information in the three-dimensional ultrasound image on the display device, the envelope information being used for indicating the to-be-detected object.

The processor 201 is further configured to perform the following steps: acquiring a first hypercomplex signal corresponding to the three-dimensional radio-frequency signal, the first hypercomplex signal being a sum of 8 components, and each of the 8 components being represented by modulus values and angles of multiple analytic signals corresponding to the input signal; and acquiring a modulus value of the first hypercomplex signal, the modulus value of the first hypercomplex signal being used for representing the three-dimensional amplitude of the three-dimensional radio-frequency signal, and envelope information including the modulus value of the first hypercomplex signal.

The processor 201 is further configured to perform the following steps: acquiring a second hypercomplex signal corresponding to the three-dimensional radio-frequency signal, the second hypercomplex signal including 8 components, and each of the 8 components being represented by the Hilbert transform of the input signal; acquiring a correspondence between the components represented by the Hilbert transform and the modulus values and angles of the multiple analytic signals; and transforming the second hypercomplex signal into the first hypercomplex signal according to the correspondence.

The processor 201 is further configured to perform the following step: acquiring the modulus value of the first hypercomplex signal according to the following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

where $|\psi_{cas}|$ represents the modulus value of the first hypercomplex signal, $\alpha_1$ is a modulus value of a first analytic signal, $\varphi_1$ is an angle of the first analytic signal, the first analytic signal is an analytic signal that is in the first orthant of 8 orthants in a three-dimensional frequency domain and that corresponds to the input signal, $\alpha_3$ is a modulus value of a third analytic signal, $\varphi_3$ is an angle of the third analytic signal, the third analytic signal is an analytic signal that is in the third orthant of the 8 orthants in the three-dimensional frequency domain and that corresponds to the input signal, $\alpha_5$ is a modulus value of a fifth analytic signal, $\varphi_5$ is an angle of the fifth analytic signal, the fifth analytic signal is an analytic signal that is in the fifth orthant of the 8 orthants in the three-dimensional frequency domain and that corresponds to the input signal, $\alpha_7$ is a modulus value of a seventh analytic signal, $\varphi_7$ is an angle of the seventh analytic signal, the seventh analytic signal is an analytic signal that is in the seventh orthant of the 8 orthants in the three-dimensional frequency domain and that corresponds to the input signal, and the multiple analytic signals include the first analytic signal, the third analytic signal, the fifth analytic signal, and the seventh analytic signal.

Optionally, for a specific example in this embodiment, reference may be made to the example described in the foregoing embodiment, and details are not described herein again in this embodiment.

A solution for displaying an ultrasound image is provided according to the embodiments of the present disclosure. By acquiring the input signal obtained by performing the detection on the to-be-detected object by the detection device, the input signal being the three-dimensional radio-frequency signal; performing the one-time modulus value calculation on the three-dimensional radio-frequency signal to obtain the envelope information in the three-dimensional ultrasound image, the one-time modulus value calculation being at least used for directly acquiring the three-dimensional amplitude of the three-dimensional radio-frequency signal; and displaying the envelope information in the three-dimensional ultrasound image on the display device, the envelope information being used for indicating the to-be-detected object, the to-be-detected object is accurately displayed in the three-dimensional ultrasound image, to achieve a technical effect of improving the accuracy of the three-dimensional ultrasound image, thereby solving the technical problem that the three-dimensional B-mode ultrasound image reconstructed in the related art has a reconstruction error that reduces the accuracy of the three-dimensional B-mode ultrasound image.

According to still another aspect of the embodiments of the present disclosure, a storage medium is further provided. The storage medium stores a computer program, the computer program being configured to perform a step of a method for displaying an ultrasound image in the foregoing embodiment when being run.

Optionally, in this embodiment, the storage medium may be located in at least one network device of multiple network devices in a network shown in the foregoing embodiments.

Optionally, in this embodiment, the storage medium is configured to store the computer program for performing the following steps S1 to S3.

In step S1, an input signal obtained by performing detection on a to-be-detected object by a detection device is acuqired, the input signal being a three-dimensional radio-frequency signal.

In step S2, a one-time modulus value calculation is performed on the three-dimensional radio-frequency signal to obtain envelope information in a three-dimensional ultrasound image, the one-time modulus value calculation being at least used for directly acquiring a three-dimensional amplitude of the three-dimensional radio-frequency signal.

In step S3, the envelope information in the three-dimensional ultrasound image is displayed on a display device, the envelope information being used for indicating the to-be-detected object.

Optionally, the storage medium is further configured to store the computer program for performing the following steps: acquiring a first hypercomplex signal corresponding to the three-dimensional radio-frequency signal, the first hypercomplex signal being a sum of 8 components, and each of the 8 components being represented by modulus values and angles of multiple analytic signals corresponding to the input signal; and acquiring a modulus value of the first hypercomplex signal, the modulus value of the first hypercomplex signal being used for representing the three-dimensional amplitude of the three-dimensional radio-frequency signal, and envelope information including the modulus value of the first hypercomplex signal.

Optionally, the storage medium is further configured to store the computer program for performing the following steps: acquiring a second hypercomplex signal corresponding to the three-dimensional radio-frequency signal, the second hypercomplex signal including 8 components, and each of the 8 components being represented by the Hilbert transform of the input signal; acquiring a correspondence between the components represented by the Hilbert transform and the modulus values and angles of the multiple analytic signals; and transforming the second hypercomplex signal into the first hypercomplex signal according to the correspondence.

The storage medium is further configured to store the computer program for performing the following step: acquiring the modulus value of the first hypercomplex signal according to the following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

where $|\psi_{cas}|$ represents the modulus value of the first hypercomplex signal, $a_1$ is a modulus value of a first analytic signal, $\varphi_1$ is an angle of the first analytic signal, the first analytic signal is an analytic signal that is in the first orthant of 8 orthants in a three-dimensional frequency domain and that corresponds to the input signal, $a_3$ is a modulus value of a third analytic signal, $\varphi_3$ is an angle of the third analytic signal, the third analytic signal is an analytic signal that is in the third orthant of the 8 orthants in the three-dimensional frequency domain and that corresponds to the input signal, $a_5$ is a modulus value of a fifth analytic signal, $\varphi_5$ is an angle of the fifth analytic signal, the fifth analytic signal is an analytic signal that is in the fifth orthant of the 8 orthants in the three-dimensional frequency domain and that corresponds to the input signal, $a_7$ is a modulus value of a seventh analytic signal, $\varphi_7$ is an angle of the seventh analytic signal, the seventh analytic signal is an analytic signal that is in the seventh orthant of the 8 orthants in the three-dimensional frequency domain and that corresponds to the input signal, and the multiple analytic signals include the first analytic signal, the third analytic signal, the fifth analytic signal, and the seventh analytic signal.

Optionally, for a specific example in this embodiment, reference may be made to the example described in the foregoing embodiment, and details are not described herein again in this embodiment.

Optionally, in this embodiment, a person of ordinary skill in the art may understand that all or some of the steps of the methods in the foregoing embodiments may be implemented by a program instructing relevant hardware of the terminal device. The program may be stored in a computer-readable storage medium. The storage medium may include a flash disk, a read-only memory (ROM), a random access memory (RAM), a magnetic disk, an optical disc, and the like.

The sequence numbers of the foregoing embodiments of the present disclosure are merely for the convenience of description, and do not imply the preference among the embodiments.

When the integrated unit in the foregoing embodiments is implemented in the form of a software functional unit and sold or used as an independent product, the integrated unit may be stored in the foregoing computer-readable storage medium. Based on such an understanding, the technical solution of the present disclosure essentially, or a part contributing to the related art, or all or a part of the technical solution may be implemented in a form of a software product. The computer software product is stored in a storage medium and includes several instructions for instructing one or more computer devices (which may be a personal computer, a server, a network device, or the like) to perform all or some of steps of the methods in the embodiments of the present disclosure.

In the foregoing embodiments of the present disclosure, descriptions of the embodiments have different emphases. As for parts that are not described in detail in one embodiment, reference can be made to the relevant descriptions of the other embodiments.

In the several embodiments provided in the present disclosure, it is understood that the described apparatus embodiment is merely an example. For example, the unit division is merely logical function division and may be another division in an actual implementation. For example, multiple units or components may be combined or integrated into another system, or some features may be ignored or not performed. In addition, the displayed or discussed mutual couplings or direct couplings or communication connections may be implemented by using some interfaces. The indirect couplings or communication connections between units or modules may be implemented in electric or other forms.

The units described as separate parts may or may not be physically separate. Parts displayed as units may or may not be physical units, and may be located in one position, or may be distributed on multiple network units. Some or all of the units may be selected according to actual requirements to achieve the objectives of the solutions in the embodiments.

In addition, functional units in the embodiments of the present disclosure may be integrated into one processing unit, or each of the units may exist alone physically, or two or more units are integrated into one unit. The integrated unit may be implemented in the form of hardware, or may be implemented in the form of a software function unit.

The foregoing descriptions are merely exemplary implementations of the present disclosure. A person of ordinary skill in the art may further make several improvements and refinements without departing from the scope of the claims.

**Claims**

1. A method for displaying an ultrasound image, applied to a detection device, the method comprising:

    acquiring (S202) an input signal obtained by performing detection on a to-be-detected object by the detection device, the input signal being a three-dimensional radio-frequency signal;

    performing (S204) a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and

    displaying (S206) the envelope information in the three-dimensional ultrasound image, the envelope information being used for indicating the to-be-detected object,

    wherein performing a one-time modulus value calculation on the three-dimensional radio-frequency signal comprises:

    acquiring a first hypercomplex signal corresponding to the three-dimensional radio-frequency signal, the first hypercomplex signal being a sum of 8 components, and each of the 8 components being represented by modulus values and angles of a plurality of analytic signals corresponding to the input signal; and

    acquiring the envelope information comprising a modulus value of the first hypercomplex signal, the modulus value of the first hypercomplex signal being used for representing the three-dimensional amplitude of the three-dimensional radio-frequency signal,

    wherein the modulus value of the first hypercomplex signal is acquired according to following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2},$$

    $|\psi_{cas}|$ representing the modulus value of the first hypercomplex signal, $\alpha_1$ being a modulus value of a first analytic signal, $\varphi_1$ being an angle of the first analytic signal, the first analytic signal being an analytic signal that is in a first orthant of 8 orthants in a three-dimensional frequency domain and that corresponds to the input signal, $\alpha_3$ being a modulus value of a third analytic signal, $\varphi_3$ being an angle of the third analytic signal, the third analytic signal being an analytic signal that is in a third orthant of the 8 orthants in the three-dimensional frequency domain and that corresponds to the input signal, $\alpha_5$ being a modulus value of a fifth analytic signal, $\varphi_5$ being an angle of the fifth analytic signal, the fifth analytic signal being an analytic signal that is in a fifth orthant of the 8 orthants in the three-dimensional frequency domain and that corresponds to the input signal, $\alpha_7$ being a modulus value of a seventh analytic signal, $\varphi_7$ being an angle of the seventh analytic signal, the seventh analytic signal being an analytic signal that is in a seventh orthant of the 8 orthants in the three-dimensional frequency domain and that corresponds to the input signal, and the plurality of analytic signals comprising the first analytic signal, the third analytic signal, the fifth analytic signal, and the seventh analytic signal.

2. The method according to claim 1, the acquiring a first hypercomplex signal corresponding to the three-dimensional radio-frequency signal comprising:

    acquiring a second hypercomplex signal corresponding to the three-dimensional radio-frequency signal, the second hypercomplex signal comprising 8 components, and each of the 8 components being represented by a Hilbert transform of the input signal;
acquiring a correspondence between the components represented by the Hilbert Transform and the modulus values and angles of the plurality of analytic signals; and
transforming the second hypercomplex signal into the first hypercomplex signal according to the correspondence.

3. An apparatus for displaying an ultrasound image, comprising an acquiring unit, a calculating unit and a display unit:

the acquiring unit (22) being configured to acquire an input signal obtained by performing detection on a to-be-detected object by a detection device, the input signal being a three-dimensional radio-frequency signal;
the calculating unit (24) being configured to perform a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal; and
the display unit (26) being configured to display the envelope information in the three-dimensional ultrasound image, the envelope information being used for indicating the to-be-detected object,
wherein the calculating unit comprises:

a first acquiring module, configured to acquire a first hypercomplex signal corresponding to the three-dimensional radio-frequency signal, the first hypercomplex signal being a sum of 8 components, and each of the 8 components being represented by modulus values and angles of a plurality of analytic signals corresponding to the input signal; and
a second acquiring module, configured to acquire the envelope information comprising a modulus value of the first hypercomplex signal, the modulus value of the first hypercomplex signal being used for representing the three-dimensional amplitude of the three-dimensional radio-frequency signal,
the second acquiring module being configured to acquire the modulus value of the first hypercomplex signal according to following equation:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

$|\psi_{cas}|$ representing the modulus value of the first hypercomplex signal, $\alpha_1$ being a modulus value of a first analytic signal, $\varphi_1$ being an angle of the first analytic signal, the first analytic signal being an analytic signal that is in a first orthant of 8 orthants in a three-dimensional frequency domain and that corresponds to the input signal, $\alpha_3$ being a modulus value of a third analytic signal, $\varphi_3$ being an angle of the third analytic signal, the third analytic signal being an analytic signal that is in a third orthant of the 8 orthants in the three-dimensional frequency domain and that corresponds to the input signal, $\alpha_5$ being a modulus value of a fifth analytic signal, $\varphi_5$ being an angle of the fifth analytic signal, the fifth analytic signal being an analytic signal that is in a fifth orthant of the 8 orthants in the three-dimensional frequency domain and that corresponds to the input signal, $\alpha_7$ being a modulus value of a seventh analytic signal, $\varphi_7$ being an angle of the seventh analytic signal, the seventh analytic signal being an analytic signal that is in a seventh orthant of the 8 orthants in the three-dimensional frequency domain and that corresponds to the input signal, and the plurality of analytic signals comprising the first analytic signal, the third analytic signal, the fifth analytic signal, and the seventh analytic signal.

4. The apparatus according to claim 3, the first acquiring module comprising:

a first acquiring submodule, configured to acquire a second hypercomplex signal corresponding to the three-dimensional radio-frequency signal, the second hypercomplex signal comprising 8 components, and each of the 8 components being represented by a Hilbert transform of the input signal;
a second acquiring submodule, configured to acquire a correspondence between the components represented by the Hilbert transform and the modulus values and angles of the plurality of analytic signals; and
a transforming module, configured to transform the second hypercomplex signal into the first hypercomplex signal according to the correspondence.

**Patentansprüche**

1. Verfahren zum Anzeigen eines Ultraschallbildes, angewandt auf ein Detektionsgerät, wobei das Verfahren umfasst:

Erfassen (S202) eines Eingangssignals, das durch Ausführen einer Detektion an einem zu detektierenden Objekt durch das Detektionsgerät erhalten wird, wobei das Eingangssignal ein dreidimensionales Hochfrequenzsignal ist;

Durchführen (S204) einer einmaligen Modulwertberechnung an dem dreidimensionalen Hochfrequenzsignal, um Hüllkurveninformationen in einem dreidimensionalen Ultraschallbild zu erhalten, wobei die einmalige Modulwertberechnung zumindest zum direkten Erfassen einer dreidimensionalen Amplitude des dreidimensionalen Hochfrequenzsignals verwendet wird; und

Anzeigen (S206) der Hüllkurveninformation in dem dreidimensionalen Ultraschallbild, wobei die Hüllkurveninformation zum Zeigen des zu detektierenden Objekts verwendet wird,

wobei das Durchführen einer einmaligen Modulwertberechnung an dem dreidimensionalen Hochfrequenzsignal umfasst:

Erfassen eines ersten hyperkomplexen Signals, das zu dem dreidimensionalen Hochfrequenzsignal korrespondiert, wobei das erste hyperkomplexe Signal eine Summe von 8 Komponenten ist und jede der 8 Komponenten durch Modulwerte und Winkel einer Mehrzahl von analytischen Signalen, die zu dem Eingangssignal korrespondieren, dargestellt wird; und

Erfassen der Hüllkurveninformation, die einem Modulwert des ersten hyperkomplexen Signals umfasst, wobei der Modulwert des ersten hyperkomplexen Signals zum Repräsentieren der dreidimensionalen Amplitude des dreidimensionalen Hochfrequenzsignals verwendet wird,

wobei der Modulwert des ersten hyperkomplexen Signals gemäß der folgenden Gleichung erfasst wird:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

,

wobei $|\psi_{cas}|$ den Modulwert des ersten hyperkomplexen Signals repräsentiert, wobei $\alpha_1$ ein Modulwert eines ersten analytischen Signals ist, wobei $\varphi_1$ ein Winkel des ersten analytischen Signals ist, wobei das erste analytische Signal ein analytisches Signal ist, das in einem ersten Orthanten von 8 Orthanten in einem dreidimensionalen Frequenzbereich liegt und das zu dem Eingangssignal korrespondiert, wobei $\alpha_3$ ein Modulwert eines dritten analytischen Signals ist, wobei $\varphi_3$ ein Winkel des dritten analytischen Signals ist, wobei das dritte analytische Signal ein analytisches Signal ist, das in einem dritten Orthanten der 8 Orthanten in dem dreidimensionalen Frequenzbereich liegt und das zu dem Eingangssignal korrespondiert, wobei $\alpha_5$ ein Modulwert eines fünften analytischen Signals ist, wobei $\varphi_5$ ein Winkel des fünften analytischen Signals ist, wobei das fünfte analytische Signal ein analytisches Signal ist, das in einem fünften Orthanten der 8 Orthanten in dem dreidimensionalen Frequenzbereich liegt und das dem Eingangssignal entspricht, wobei $\alpha_7$ ein Modulwert eines siebten analytischen Signals ist, wobei $\varphi_7$ ein Winkel des siebten analytischen Signals ist, wobei das siebte analytische Signal ein analytisches Signal ist, das in einem siebten Orthanten der 8 Orthanten in dem dreidimensionalen Frequenzbereich liegt und das zu dem Eingangssignal korrespondiert, und wobei die Mehrzahl von analytischen Signalen das erste analytische Signal, das dritte analytische Signal, das fünfte analytische Signal und das siebte analytische Signal umfasst.

2. Verfahren nach Anspruch 1, wobei das Erfassen eines ersten hyperkomplexen Signals, das zu dem dreidimensionalen Hochfrequenzsignal korrespondiert, umfasst:

Erfassen eines zweiten hyperkomplexen Signals, das zu dem dreidimensionalen Hochfrequenzsignal korrespondiert, wobei das zweite hyperkomplexe Signal 8 Komponenten umfasst und jede der 8 Komponenten durch eine Hilbert-Transformation des Eingangssignals repräsentiert wird;

Erfassen einer Korrespondenz zwischen den durch die Hilbert-Transformation repräsentierten Komponenten und den Modulwerten und Winkeln der Mehrzahl von analytischen Signalen; und

Transformieren des zweiten hyperkomplexen Signals in das erste hyperkomplexe Signal entsprechend der Korrespondenz.

3. Vorrichtung zum Anzeigen eines Ultraschallbildes, die eine Erfassungseinheit, eine Berechnungseinheit und eine Anzeigeeinheit umfasst, wobei:

die Erfassungseinheit (22) konfiguriert ist zum Erfassen eines Eingangssignals, das durch Ausführen einer Detektion an einem zu detektierenden Objekt durch ein Detektionsgerät erhalten wird, wobei das Eingangssignal ein dreidimensionales Hochfrequenzsignal ist;
die Berechnungseinheit (24) konfiguriert ist zum Durchführen einer einmaligen Modulwertberechnung an dem dreidimensionalen Hochfrequenzsignal, um Hüllkurveninformationen in einem dreidimensionalen Ultraschallbild zu erhalten, wobei die einmalige Modulwertberechnung zumindest zum direkten Erfassen einer dreidimensionalen Amplitude des dreidimensionalen Hochfrequenzsignals verwendet wird; und
die Anzeigeeinheit (26) konfiguriert ist zum Anzeigen der Hüllkurveninformation in dem dreidimensionalen Ultraschallbild, wobei die Hüllkurveninformation zum Zeigen des zu detektierenden Objekts verwendet wird, wobei die Berechnungseinheit umfasst:

ein erstes Erfassungsmodul, das konfiguriert ist zum Erfassen eines ersten hyperkomplexen Signals, das zu dem dreidimensionalen Hochfrequenzsignal korrespondiert, wobei das erste hyperkomplexe Signal eine Summe von 8 Komponenten ist und jede der 8 Komponenten durch Modulwerte und Winkel einer Mehrzahl von analytischen Signalen, die zu dem Eingangssignal korrespondieren, dargestellt wird; und
ein zweites Erfassungsmodul, das konfiguriert ist zum Erfassen der Hüllkurveninformation, die einem Modulwert des ersten hyperkomplexen Signals umfasst, wobei der Modulwert des ersten hyperkomplexen Signals zum Repräsentieren der dreidimensionalen Amplitude des dreidimensionalen Hochfrequenzsignals verwendet wird,
wobei das zweite Erfassungsmodul konfiguriert ist zum Erfassen des Modulwerts des ersten hyperkomplexen Signals gemäß der folgenden Gleichung:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

wobei $|\psi_{cas}|$ den Modulwert des ersten hyperkomplexen Signals repräsentiert, wobei $\alpha_1$ ein Modulwert eines ersten analytischen Signals ist, wobei $\varphi_1$ ein Winkel des ersten analytischen Signals ist, wobei das erste analytische Signal ein analytisches Signal ist, das in einem ersten Orthanten von 8 Orthanten in einem dreidimensionalen Frequenzbereich liegt und das zu dem Eingangssignal korrespondiert, wobei $\alpha_3$ ein Modulwert eines dritten analytischen Signals ist, wobei $\varphi_3$ ein Winkel des dritten analytischen Signals ist, wobei das dritte analytische Signal ein analytisches Signal ist, das in einem dritten Orthanten der 8 Orthanten in dem dreidimensionalen Frequenzbereich liegt und das zu dem Eingangssignal korrespondiert, wobei $\alpha_5$ ein Modulwert eines fünften analytischen Signals ist, wobei $\varphi_5$ ein Winkel des fünften analytischen Signals ist, wobei das fünfte analytische Signal ein analytisches Signal ist, das in einem fünften Orthanten der 8 Orthanten in dem dreidimensionalen Frequenzbereich liegt und das dem Eingangssignal entspricht, wobei $\alpha_7$ ein Modulwert eines siebten analytischen Signals ist, wobei $\varphi_7$ ein Winkel des siebten analytischen Signals ist, wobei das siebte analytische Signal ein analytisches Signal ist, das in einem siebten Orthanten der 8 Orthanten in dem dreidimensionalen Frequenzbereich liegt und das zu dem Eingangssignal korrespondiert, und wobei die Mehrzahl von analytischen Signalen das erste analytische Signal, das dritte analytische Signal, das fünfte analytische Signal und das siebte analytische Signal umfasst.

4. Vorrichtung nach Anspruch 3, wobei das erste Erfassungsmodul umfasst:

ein erstes Erfassungs-Submodul, das konfiguriert ist zum Erfassen eines zweiten hyperkomplexen Signals, das zu dem dreidimensionalen Hochfrequenzsignal korrespondiert, wobei das zweite hyperkomplexe Signal 8 Komponenten umfasst und jede der 8 Komponenten durch eine Hilbert-Transformation des Eingangssignals repräsentiert wird;
ein zweites Erfassungs-Submodul, das konfiguriert ist zum Erfassen einer Korrespondenz zwischen den durch die Hilbert-Transformation repräsentierten Komponenten und den Modulwerten und Winkeln der Mehrzahl von analytischen Signalen; und
ein Transformationsmodul, das konfiguriert ist zum Transformieren des zweiten hyperkomplexen Signals in das erste hyperkomplexe Signal entsprechend der Korrespondenz.

**Revendications**

1. Procédé d'affichage d'une image ultrasonore, appliqué à un dispositif de détection, le procédé comprenant:

   acquérir (S202) un signal d'entrée obtenu en effectuant une détection sur un objet à détecter par le dispositif de détection, le signal d'entrée étant un signal radiofréquence tridimensionnel,

   effectuer (S204) un calcul de valeur de module unique sur le signal radiofréquence tridimensionnel pour obtenir des informations d'enveloppe dans une image ultrasonore tridimensionnelle, le calcul de valeur de module unique étant au moins utilisé pour acquérir directement une amplitude tridimensionnelle du signal radiofréquence tridimensionnel, et

   afficher (S206) les informations d'enveloppe dans l'image ultrasonore tridimensionnelle, les informations d'enveloppe étant utilisées pour indiquer l'objet à détecter,

   dans lequel la mise en oeuvre d'un calcul de valeur de module unique sur le signal radiofréquence tridimensionnel comprend:

   acquérir un premier signal hypercomplexe correspondant au signal radiofréquence tridimensionnel, le premier signal hypercomplexe étant une somme de 8 composantes, et chacune des 8 composantes étant représentée par des valeurs de module et des angles d'une pluralité de signaux analytiques correspondant au signal d'entrée, et

   acquérir les informations d'enveloppe comprenant une valeur de module du premier signal hypercomplexe, la valeur de module du premier signal hypercomplexe étant utilisée pour représenter l'amplitude tridimensionnelle du signal radiofréquence tridimensionnel,

   dans lequel la valeur de module du premier signal hypercomplexe est acquise selon l'équation suivante,

   $$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

   ,

   $|\psi_{cas}|$ représentant la valeur de module du premier signal hypercomplexe, $a_1$ étant une valeur de module d'un premier signal analytique, $\varphi_1$ étant un angle du premier signal analytique, le premier signal analytique étant un signal analytique qui est dans un premier orthant de 8 orthants dans un domaine fréquentiel tridimensionnel et qui correspond au signal d'entrée, $a_3$ étant une valeur de module d'un troisième signal analytique, $\varphi_3$ étant un angle du troisième signal analytique, le troisième signal analytique étant un signal analytique qui se trouve dans un troisième orthant des 8 orthants dans le domaine fréquentiel tridimensionnel et qui correspond au signal d'entrée, $a_5$ étant une valeur de module d'un cinquième signal analytique, $\varphi_5$ étant un angle du cinquième signal analytique, le cinquième signal analytique étant un signal analytique qui est dans un cinquième orthant des 8 orthants dans le domaine fréquentiel tridimensionnel et qui correspond au signal d'entrée, $a_7$ étant une valeur de module d'un septième signal analytique, $\varphi_7$ étant un angle du septième signal analytique, le septième signal analytique étant un signal analytique qui est dans un septième orthant des 8 orthants dans le domaine fréquentiel tridimensionnel et qui correspond au signal d'entrée, et la pluralité de signaux analytiques comprenant le premier signal analytique, le troisième signal analytique, le cinquième signal analytique et le septième signal analytique.

2. Procédé selon la revendication 1, l'acquisition d'un premier signal hypercomplexe correspondant au signal radiofréquence tridimensionnel comprenant:

   acquérir un deuxième signal hypercomplexe correspondant au signal radiofréquence tridimensionnel, le deuxième signal hypercomplexe comprenant 8 composantes, et chacune des 8 composantes étant représentée par une transformée de Hilbert du signal d'entrée,

   acquérir une correspondance entre les composantes représentées par la transformée de Hilbert et les valeurs de module et les angles de la pluralité de signaux analytiques, et

   transformer le deuxième signal hypercomplexe en ledit premier signal hypercomplexe selon la correspondance.

3. Dispositif d'affichage d'une image ultrasonore, comprenant une unité d'acquisition, une unité de calcul et une unité d'affichage:

l'unité d'acquisition (22) étant configurée pour acquérir un signal d'entrée obtenu en effectuant une détection sur un objet à détecter par un dispositif de détection, le signal d'entrée étant un signal radiofréquence tridimensionnel,

l'unité de calcul (24) étant configurée pour effectuer un calcul de valeur de module unique sur le signal radiofréquence tridimensionnel pour obtenir des informations d'enveloppe dans une image ultrasonore tridimensionnelle, le calcul de valeur de module unique étant au moins utilisé pour acquérir directement une amplitude tridimensionnelle du signal radiofréquence tridimensionnel, et

l'unité d'affichage (26) étant configurée pour afficher les informations d'enveloppe dans l'image ultrasonore tridimensionnelle, les informations d'enveloppe étant utilisées pour indiquer l'objet à détecter,

dans lequel l'unité de calcul comprend:

un premier module d'acquisition configuré pour acquérir un premier signal hypercomplexe correspondant au signal radiofréquence tridimensionnel, le premier signal hypercomplexe étant une somme de 8 composantes, et chacune des 8 composantes étant représentée par des valeurs de module et des angles d'une pluralité de signaux analytiques correspondant au signal d'entrée, et

un deuxième module d'acquisition configuré pour acquérir les informations d'enveloppe comprenant une valeur de module du premier signal hypercomplexe, la valeur de module du premier signal hypercomplexe étant utilisée pour représenter l'amplitude tridimensionnelle du signal radiofréquence tridimensionnel,

le deuxième module d'acquisition étant configuré pour acquérir la valeur de module du premier signal hypercomplexe selon l'équation suivante:

$$|\psi_{cas}| = \sqrt[4]{\left[\frac{a_1^2 + a_3^2 + a_5^2 + a_7^2}{4}\right]^2 - \left[\frac{a_1 a_3 \sin(\varphi_1 - \varphi_3) - a_5 a_7 \sin(\varphi_5 - \varphi_7)}{2}\right]^2}$$

$|\psi_{cas}|$ représentant la valeur de module du premier signal hypercomplexe, $\alpha_1$ étant une valeur de module d'un premier signal analytique, $\varphi_1$ étant un angle du premier signal analytique, le premier signal analytique étant un signal analytique qui est dans un premier orthant de 8 orthants dans un domaine fréquentiel tridimensionnel et qui correspond au signal d'entrée, $\alpha_3$ étant une valeur de module d'un troisième signal analytique, $\varphi_3$ étant un angle du troisième signal analytique, le troisième signal analytique étant un signal analytique qui se trouve dans un troisième orthant des 8 orthants dans le domaine fréquentiel tridimensionnel et qui correspond au signal d'entrée, $\alpha_5$ étant une valeur de module d'un cinquième signal analytique, $\varphi_5$ étant un angle du cinquième signal analytique, le cinquième signal analytique étant un signal analytique qui est dans un cinquième orthant des 8 orthants dans le domaine fréquentiel tridimensionnel et qui correspond au signal d'entrée, $\alpha_7$ étant une valeur de module d'un septième signal analytique, $\varphi_7$ étant un angle du septième signal analytique, le septième signal analytique étant un signal analytique qui est dans un septième orthant des 8 orthants dans le domaine fréquentiel tridimensionnel et qui correspond au signal d'entrée, et la pluralité de signaux analytiques comprenant le premier signal analytique, le troisième signal analytique, le cinquième signal analytique et le septième signal analytique.

4. Dispositif selon la revendication 3, le premier module d'acquisition comprenant:

un premier sous-module d'acquisition configuré pour acquérir un deuxième signal hypercomplexe correspondant au signal radiofréquence tridimensionnel, le deuxième signal hypercomplexe comprenant 8 composantes, et chacune des 8 composantes étant représentée par une transformée de Hilbert du signal d'entrée,

un deuxième sous-module d'acquisition configuré pour acquérir une correspondance entre les composantes représentées par la transformée de Hilbert et les valeurs de module et les angles de la pluralité de signaux analytiques, et

un module de transformation configuré pour transformer le deuxième signal hypercomplexe en ledit premier signal hypercomplexe selon la correspondance.

**FIG. 1**

Acquire an input signal obtained by performing detection on a to-be-detected object by a detection device, the input signal being a three-dimensional radio-frequency signal — S202

Perform a one-time modulus value calculation on the three-dimensional radio-frequency signal to obtain envelope information in a three-dimensional ultrasound image, the one-time modulus value calculation being at least used for directly acquiring a three-dimensional amplitude of the three-dimensional radio-frequency signal — S204

Display the envelope information in the three-dimensional ultrasound image, the envelope information being used for indicating the to-be-detected object — S206

**FIG. 2**

z axis

Three-dimensional
ultrasound fan-shaped probe

x axis

y axis

High-frequency
signal f(x)

**FIG. 3**

**FIG. 4**

Three-dimensional
fan-shaped ultrasound probe

Biopsy needle

Phantom

**FIG. 5**

Elevation

Lateral

Axial

Biopsy needle

Slice 33

Slice 15

Slice 1

**FIG. 6**

**FIG. 7**

**FIG. 8**

**FIG. 9**

**FIG. 10**

Linear ultrasound probe

Biopsy needle

Phantom

**FIG. 11**

FIG. 12

FIG. 13

FIG. 14

**FIG. 15**

Acquiring unit 22

Calculating unit 24

Display unit 26

**FIG. 16**

Input/output device 207

Transmission apparatus 205

Processor 201

Memory 203

**FIG. 17**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201810508663
- US 2016238568 A1
- CN 101061961 A
- CN 103512960 A

**Non-patent literature cited in the description**

- **L. WANG et al.** 3-D biquaternionic analytic signal and application to envelope detection in 3-D ultrasound imaging. *IEEE International Conference on 3D Imaging,* 2012
- **K.M. SNOPEK.** The n-D Analytic Signals and Fourier Spectra in Complex and Hypercomplex Domains. *IEEE International Conference on Telecommunications and Signal Processing,* 2011
- **T. BULOW et al.** Hypercomplex Signals-A Novel Extension of the Analytic Signal to the Multidimensional Case. *IEEE Transactions on Signal Processing,* 2001